# EUROPEAN PATENT APPLICATION

(11) **EP 2 289 507 A1**
(43) Date of publication of application: **02.03.2011**
(21) Application number: 10179280.2
(22) Date of filing: 17.03.2004
(51) Int. Cl.: A61K 31/265

(54) **Pharmaceutical compositions of CETP inhibitors**

(30) Priority: 17.03.2003 US 455293 P; 04.04.2003 US 460521 P; 10.06.2003 US 477202 P; 08.08.2003 US 493649 P
(62) Divisional of application: 04721345.9
(71) Applicant: Japan Tobacco, Inc., Tokyo 105-8422 (JP)
(72) Inventor: Sunami, Masaki, Takatsuki-shi Osaka 5691125 (JP); Serigano, Takanori, Takatsuki-shi Osaka 5691125 (JP)
(74) Representative: Vossius & Partner

(57) **Abstract**

The invention provides pharmaceutical compositions comprising a cholesteryl ester transfer protein (CEPT) inhibitor and a water-insoluble concentration-enhancing additive, which exhibit improved bioavailability. The invention also provides methods of treating cardiovascular disorders comprising the administration of the pharmaceutical compositions comprising a CETP inhibitor.

## Description

### TECHNICAL FIELD

This invention pertains to compositions and methods for the treatment or prophylaxis of cardiovascular disorders comprising CETP inhibitors.

### BACKGROUND ART

Hyperlipidemic conditions associated with elevated concentrations of total cholesterol and low-density lipoprotein (LDL) cholesterol are major risk factors for coronary heart disease, and atherosclerosis in particular. Additionally, numerous studies have demonstrated that a low plasma concentration of high-density lipoprotein (HDL) cholesterol is a powerful risk factor for the development of atherosclerosis.

Cholesteryl ester transfer protein (CETP) is a plasma protein that facilitates the movement of cholesteryl esters and triglycerides between various lipoproteins in the blood. The movement of cholesteryl ester from HDL to LDL by CETP has the effect of lowering HDL cholesterol and increasing LDL cholesterol. Inhibition of CETP activity by CETP inhibitors has been shown to effectively modify plasmid HDL/LDL ratios by elevating plasma HDL cholesterol and lowering plasma LDL cholesterol.

To be effective, CETP inhibitors must be absorbed into the blood. Oral dosing of CETP inhibitors is preferred because to be effective such CETP inhibitors must be taken on a regular basis, such as daily. CETP inhibitors, particularly those that have high binding activity, are generally hydrophobic, have extremely low aqueous solubility, and have low oral bioavailability when dosed conventionally. Such compounds have generally proven to be difficult to formulate for oral administration such that high bioavailabilities are achieved.

International Patent Application WO 02/11710 recognizes this problem of low bioavailability and attempts to solve such a problem by formulating a composition comprising a solid dispersion of a CETP inhibitor in an amorphous form and a watersoluble polymer that increases the concentration of the CETP inhibitor in the environment of use. However, as many CETP inhibitors are in crystalline form, there is an ongoing need for improved compositions of crystalline CETP inhibitors.

Therefore, there remains a need for pharmaceutical compositions comprising CETP inhibitors in crystalline form that result in increased bioavailabilty of the CETP inhibitors in the environment of use. The invention provides such a pharmaceutical composition and methods of treating cardiovascular disorders using the pharmaceutical compositions. These and other advantages of the invention, as well as additional inventive features, will be apparent from the description of the invention provided herein.

### DISCLOSURE OF INVENTION

The invention provides a pharmaceutical composition comprising a cholesteryl ester transfer protein inhibitor and a water-insoluble concentration-enhancing additive, such as crospovidone.

The invention also provides a method for treating or preventing a cardiovascular disorder in a mammal by administering to a mammal in need of such treatment a therapeutically effective amount of the pharmaceutical composition provided by the invention.

### BRIEF DESCRIPTION OF DRAWINGS

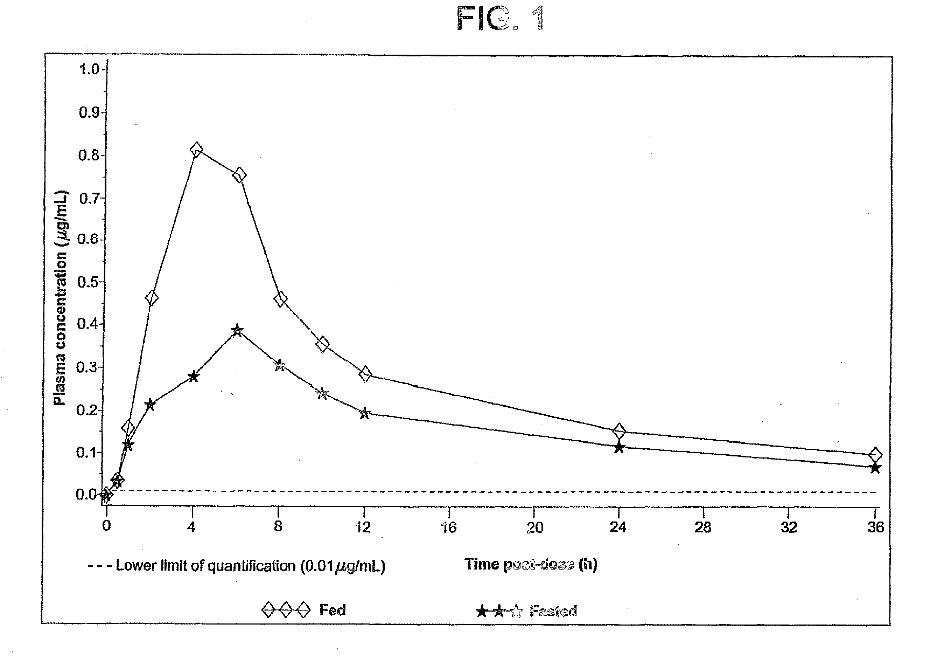

Figure 1 is a linear plot of the geometric mean plasma concentrations (µg/mL) of the active form of *S*-[2-([[1-(2-ethylbutyl)cyclohexyl]carbonyl]amino)phenyl] 2-methylpropanethioate over 36 hours in Caucasian male patients, who were orally administered 900 mg of *S*-[2-([[1-(2-ethylbutyl)cyclohexyl]carbonyl]amino)phenyl] 2-methylpropanethioate with food or without food.

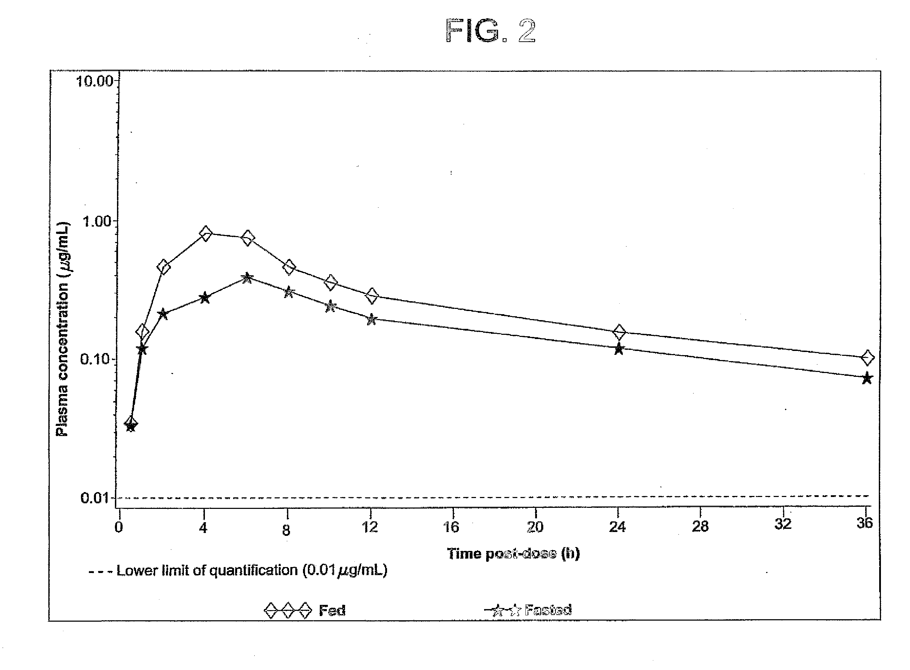

Figure 2 is a semi-logarithmic plot of the geometric mean plasma concentrations (µg/mL) of the active form of *S*-[2-([[1-(2-ethylbutyl)cyclohexyl]carbonyl]amino)phenyl] 2-methylpropanethioate over 36 hours in Caucasian male patients, who were orally administered 900 mg of *S*-[2-([[1-(2-ethylbutyl)cyclohexyl]carbonyl]amino)phenyl] 2-methylpropanethioate with food or without food.

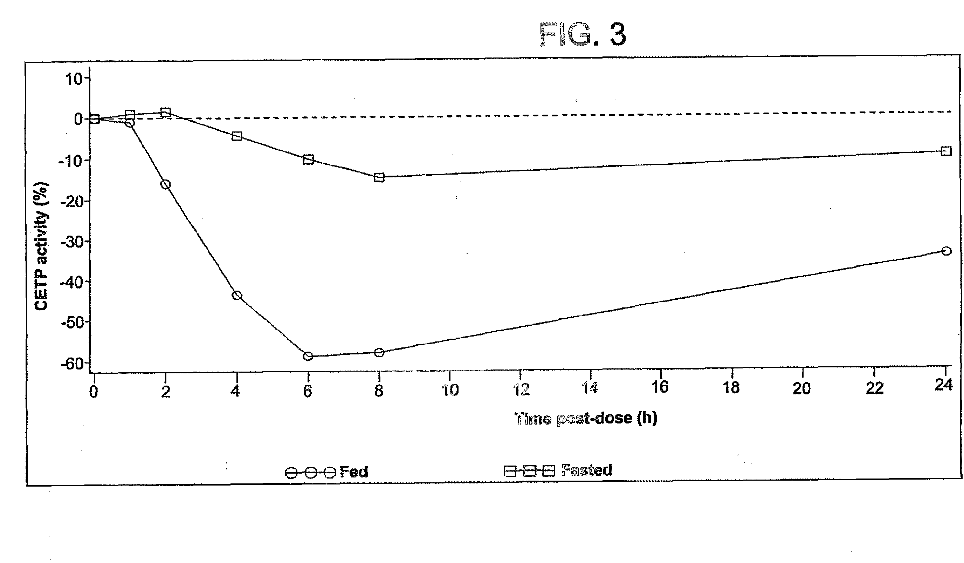

Figure 3 is plot of the mean changes from baseline (pre-dose) in CETP activity over 24 hours in Caucasian male patients, who were orally administered 900 mg of *S*-[2-([[1-(2-ethylbutyl)cyclohexyl]carbonyl]amino)phenyl] 2-methylpropanethioate with food or without food.

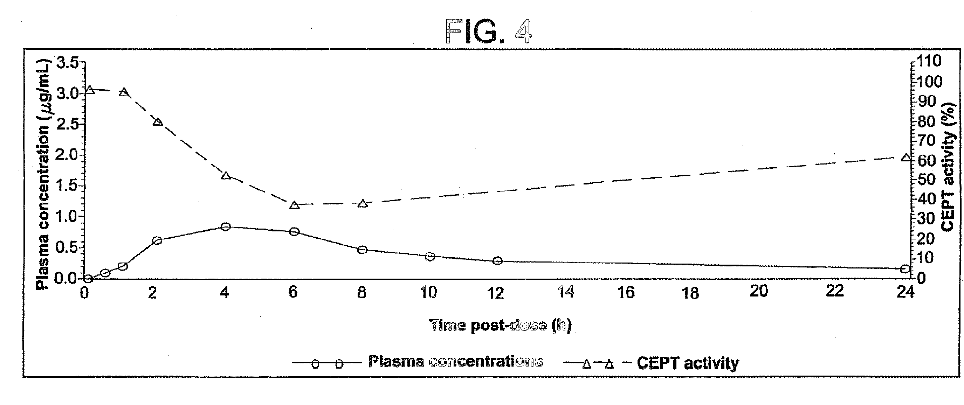

Figure 4 is a plot of mean CETP activities and mean plasma concentrations of the active form of *S*-[2-([[1-(2-ethylbutyl)cyclohexyl]carbonyl]amino)phenyl] 2-methylpropanethioate over 24 hours in Caucasian male patients following the oral administration of 900 mg of *S*-[2-([[1-(2-ethylbutyl)cyclohexyl]carbonyl]amino)phenyl] 2-methylpropanethioate with food.

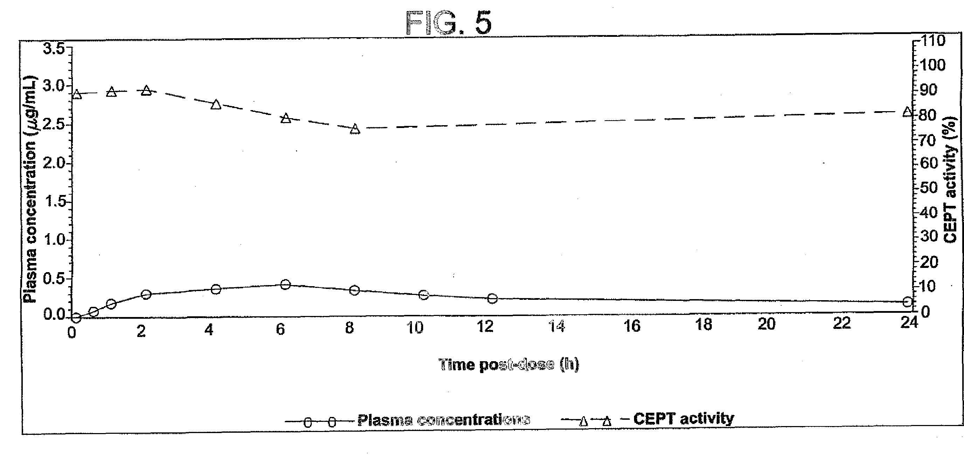

Figure 5 is a plot of mean CETP activities and mean plasma concentrations of the active form of *S*-[2-([[1-(2-ethylbutyl)cyclohexyl]carbonyl]amino)phenyl] 2-methylpropanethioate over 24 hours in Caucasian male patients following the oral administration of 900 mg of *S*-[2-([[1-(2-ethylbutyl)cyclohexyl]carbonyl]amino)phenyl] 2-methylpropanethioate without food.

The invention provides compositions of at least one CETP inhibitor and at least one water-insoluble concentration-enhancing additive, wherein the additive desirably increases the bioavailability of the CETP inhibitor, or the active form thereof, relative to the administration of the CETP inhibitor in the absence of the additive.

The CETP inhibitor signifies any compound that inhibits CETP or forms an active form that inhibits CETP. Any suitable CETP inhibitor can be used in the context of the invention, such as those described in U.S. Patents 6,140,342, 6,140,343, 6,147,089, 6,147,090,6,197,786, and 6,426,365; European Patent Application Numbers EP 796846 Al and EP 818448 Al; and International Patent Application Numbers WO 98/04528, WO 98/35937, WO 99/14174, WO 99/14204, WO 99/14215, WO 99/41237, and WO 02/11710.

Preferably, the CETP inhibitor is a compound of Formula I: 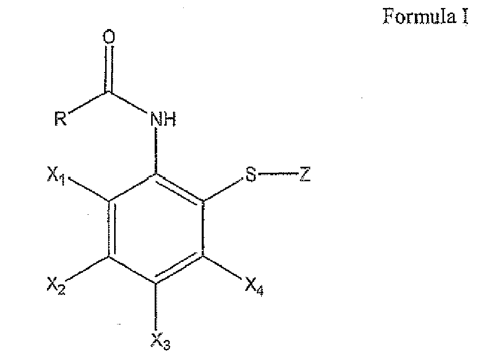 or a prodrug compound, pharmaceutically acceptable salt, enantiomer, stereoisomer, hydrate, or solvate of the compound of Formula I. In Formula I, R represents a substituted or unsubstituted C₃₋₁₀ cycloalkyl group or a substituted or unsubstituted C₅₋₈ cycloalkenyl group. Each of X₁, X₂, X₃, and X₄ may be the same or different and represents one or more of the following: a hydrogen atom; a halogen atom; a C₁₋₄ alkyl group; a halo-C₁₋₄ alkyl group; a C₁₋₄ alkoxy group; a cyano group; a nitro group; an acyl group; or an aryl group. Z represents a hydrogen atom, -YR₁ (wherein Y represents -CO- or -CS-, and R₁ represents a substituted or unsubstituted straight chain or branched C₁₋₁₀ alkyl group; a C₁₋₄ alkoxy group; a C₁₋₄ alkylthio group; a substituted or unsubstituted amino group; a substituted or unsubstituted ureido group; a substituted or unsubstituted C₃₋₁₀ cycloalkyl group; a substituted or unsubstituted C₃₋₁₀ cycloalkyl C₁₋₁₀ alkyl group; a substituted or unsubstitued aryl group; a substituted or unsubstituted aralkyl group; a substituted or unsubstituted arylalkenyl group; a substituted or unsubstituted arylthio group; a substituted or unsubstituted 5- or 6-membered heterocyclic group having 1-3 nitrogen, oxygen, or sulfur atoms; or a substituted or unsubstituted 5- or 6-membered heteroarylahlyl group), or -S-R₂ (wherein R₂ represents a substituted or unsubstituted C₁₋₄ alkyl group or a substituted or unsubstituted aryl group).

The term "straight chain or branched C₁₋₁₀ alkyl group" used herein means an alkyl group having 1-10 carbon atoms which may be straight or branched. Specific examples thereof include methyl, ethyl, propyl, isopropyl, butyl, isobutyl, sec-butyl, tert-butyl, pentyl, isopentyl, neopentyl, tert-pentyl, 1-ethylbutyl, 2-ethylbutyl, 1-propylbutyl, 1,1-dimethylbutyl, 1-isobutyl-3-methylbutyl, 1-ethylpentyl, 1-propylpentyl, 1-isobutylpentyl, 2-ethylpentyl, 2-isopropylpentyl, 2-tert-butylpentyl, 3-ethylpentyl, 3-isopropylpentyl, 4-methylpentyl, 1,4-dimethylpentyl, 2,4-dimethylpentyl, 1-ethyl-4-methylpentyl, hexyl, 1-ethylhexyl, 1-propylhexyl, 2-ethylhexyl, 2-isopropylhexyl, 2-tert-butylhexyl, 3-ethylhexyl, 3-isopropylhexyl, 3-tert-butylhexyl, 4-ethylhexyl, 5-methylhexyl, heptyl, 1-ethytheptyl, 1-isopropylheptyl, 2-ethylheptyl, 2-isopropylheptyl, 3-propylheptyl, 4-propylheptyl, 5-ethylheptyl, 6-methylheptyl, octyl, 1-ethyloctyl, 2-ethyloctyl, nonyl, 1-methylnonyl, 2-methylnonyl, decyl, and the like groups. A straight chain or branched alkyl group having 1-8 carbon atoms is preferred.

The term "C₁₋₄ lower alkyl group" used herein means an alkyl group having 1-4 carbon atoms, and specifically includes methyl, ethyl, propyl, isopropyl, butyl, isobutyl, see-butyl, tert-butyl, and the like groups.

The term "straight chain or branched C₂₋₁₀ alkenyl group" means an alkenyl group having 2-10 carbon atoms with at least one or more double bonds, which may be straight or branched. Specific examples thereof include allyl, vinyl, isopropenyl, 1-propenyl, 1-methyl-2-propenyl, 2-methyl-2-propenyl, 1-methyl-1-butenyl, crotyl, 1-methyl-3-butenyl, 3-methyl-2-butenyl, 1,3-dimethyl-2-butenyl, 1-pentenyl, 1-methyl-2-pentenyl, 1-ethyl-3-pentenyl, 4-pentenyl, 1,3-pentadienyl, 2,4-pentadienyl, 1-hexenyl, 1-methyl-2-hexenyl, 3-hexenyl, 4-hexenyl, 1-butyl-5-hexenyl, 1,3-hexadienyl, 2,4-hexadienyl, 1-heptenyl, 2-heptenyl, 3-heptenyl, 4-heptenyl, 5-heptenyl, 6-heptenyl, 1,3-heptadienyl, 2,4-heptadienyl, 1-octenyl, 2-octenyl, 3-octenyl, 4-octenyl, 5-octenyl, 6-octenyl, 7-octenyl, 1-nonenyl, 2-nonenyl, 3-nonenyl, 4-nonenyl, 5-nonenyl, 6-nonenyl, 7-nonenyl, 8-nonenyl, 9-decenyl, and the like groups. An alkenyl group having 2-8 carbon atoms, which may be straight or branched, is preferred.

The term "halogen atom" means fluorine, chlorine, and bromine atoms.

The term "halo-C₁₋₄ alkyl group" means the above-described C₁₋₄ lower alkyl group substituted with 1-3 halogens, which may be the same or different. Specific examples thereof include fluoromethyl, chloromethyl, bromomethyl, difluoromethyl, dichloromethyl, trifluoromethyl, trichloromethyl, chloroethyl, difluoroethyl, trifluoromethyl, pentachloroethyl, bromopropyl, dichloropropyl, trifluorobutyl, and the like groups. Trifluoromethyl and chloroethyl are preferred.

The term "C₁₋₄lower alkoxy group" means the alkoxy group containing the C₁₋₄ lower alkyl group as described above. Examples thereof include methoxy, ethoxy, propoxy, isopropoxy, butoxy, isobutoxy, sec-butoxy, tert-butoxy, and the like groups.

The term "C₁₋₄ lower alkylthio group" means the alkylthio group containing the C₁₋₄ lower alkyl group as described above. Examples thereof include methylthio, ethylthio, propylthio, isopropylthio, butylthio, isobutylthio, sec-butylthio, tert-butylthio, and the like groups.

The term "C₃₋₁₀ cycloalkyl group" means a cycloalkyl group having 3-10 carbon atoms, which may be monocyclic or polycyclic. Examples thereof include cyclopropyl, cyclobutyl, cyclopentyl, cyclohexyl, cycloheptyl, cyclooctyl, octahydroindenyl, decahydronaphthyl, bicyclo[2.2.1]heptyl, adamantyl, and the like groups. Preferred are those having 5-7 carbon atoms, including cyclopentyl, cyclohexyl, and cycloheptyl.

The term "C₅₋₈ cycloalkenyl group" means a cycloalkenyl group having 5-8 carbon atoms with one or more double bonds on the ring. Examples thereof include cyclopentenyl, cyclohexenyl, cycloheptenyl, cyclooctenyl, cydopentadienyl, cyclohexadienyl, cyctoheptadienyl, cyclooctadienyl, and the like groups. Preferred are those with 5-7 carbon atoms, inducing cyclopentenyl, cyctohexenyl, and cycloheptenyl.

The term "C₃₋₁₀ cycloalkyl C₁₋₁₀ alkyl group" means the above-described straight chain or branched C₁₋₁₀ alkyl group substituted with the above-described C₃₋₁₀ cycloalkyl group. Specific examples thereof include cyclopropylmethyl, cyclopentylmethyl, cyclohexylmethyl, cyclohexyl cyclopentylmethyl, dicyclohexylmethyl, 1-cyclopentylethyl, 1-cyclohexylethyl, 2-cyclopropylethyl, 2-cyclopentylethyl, 2-cyclohexylethyl, 2-cycloheptylethyl, 1-cyclohexyl-1-methylethyl, 1-cyclohexylpropyl, 2-cyclopentylpropyl, 3-cyclobutylpropyl, 3-cyclopentylpropyl, 3-cyclohexylpropyl, 3-cycloheptylpropyl, 1-cyclopropyl-1-methylpropyl, 1-cyclohexyl-2-methylpropyl, 1-cyclopentylbutyl, 1-cyclohexylbutyl, 3-cyclohexylbutyl, 4-cyclopropylbutyl, 4-cyclobutylbutyl, 4-cyclopentylbutyl, 1-cyclohexyl-1-methylbutyl, 1-cyclopentyl-2-ethylbutyl, 1-cyclohexyl-3-methylbutyl, 1-cyclopentylpentyl, 1-cyclohexylpentyl, 1-cyclohexylmethylpentyl, 2-cyclohexylpentyl, 2-cyclohexymethylpentyl, 3-cyclopentylpentyl, 1-cyclohexyl-4-methylpentyl, 5-cyclopentylpentyl, 1-cyclopentylhexyl, 1-cyclohexylhexyl, 1-cyclopentylmethylhexyl, 2-cyclopentylhexyl, 2-cyclopropylethylhexyl, 3-cyclopentylhexyl, 1-cyclohexylheptyl, 1-cyclopentyl-1-methylheptyl, 1-cyclohexyl-1,6-dimethylheptyl, 1-cycloheptyloctyl, 2-cyclopentyloctyl, 3-cyclohexyloctyl, 2-cyclopentylmethytoctyl 1-cyclopentylnonyl, 1-cyclohexylnonyl, 3-cyclopropylnonyl, 1-cyclopentyldecyl, 1-cyclohexylundecyl, 1-cyclopentyltridecyl, 2-cyclohexyltridecyl, and the like groups.

The "aryl group" includes phenyl, naphthyl, anthryl, phenanthryl, biphenyl, and the like groups. Phenyl, naphthyl and biphenyl groups are preferred.

The "aralkyl group" means the above-described C₁₋₄ lower alkyl group substituted with one or more aryl groups as described above. Examples thereof include benzyl, benzhydryl, trityl, phenethyl, 3-phenylpropyl, 2-phenylpropyl, 4-phenylbutyl, naphthylmethyl, 2-naphthylethyl, 4-biphenylmethyl, 3-(4-biphenyl) propyl, and the like groups.

The "arylalkenyl group" means an alkenyl group having 2-4 carbon atoms substituted with the above-described aryl group. Examples thereof include 2-phenylvinyl, 3-phenyl-2-propenyl, 3-phenyl-2-methyl-2-propenyl, 4-phenyl-3-butenyl, 2-(1-naphthyl)vinyl, 2-(2-naphthyl)vinyl, 2-(4-biphenyl)vinyl, and the like groups.

The "arylthio group" means an arylthio group containing the above-described aryl group and specifically include phenylthio, naphthylthio, and the like groups.

The "heterocyclic group" means a 5- and 6-membered aromatic or non-aromatic heterocyclic ring containing at least one or more, specifically 1-4, preferably 1-3, hetero atoms selected from nitrogen, oxygen, and sulfur atoms. Specific examples thereof include aromatic heterocyclic rings such as thiatriazolyl, tetrazolyl, dithiazolyl, oxadiazolyl, thiadiazolyl, triazolyl, oxazolyl, pyrazolyl, pyrrolyl, furyl, thienyl, tetrazinyl, triazinyl, pyrazinyl, pyridazinyl, pyrimidinyl, pyridyl, or the like groups and non-aromatic heterocyclic rings such as dioxoranyl, pyrrolidinyl, tetrahydrofuryl, tetrahydrothienyl, dithiadiazinyl, thiadiazinyl, morpholino, morpholinyl, oxazinyl, thiazinyl, piperazinyl, piperidyl, piperidino, pyranyl, thiopyranyl, or the like groups. Preferable groups are aromatic heterocyclic (heteroaryl) groups including furyl, thienyl, pyrrolyl, pyridyl, and the like and non-aromatic heterocyclic groups containing at least one nitrogen atom, including pyrrolidinyl, tetrahydrofuryl, piperazinyl, piperidyl, piperidino, and the like groups.

The "heteroarylalkyi group" means the above-described C₁₋₄ lower alkyl group substituted with the above-described 5- or 6-membered aromatic heterocyclic (heteroaryl) group and specifically include 2-thienylmethyl, 2-furylmethyl, 2-pyridylmethyl, 3-pyridylmethyl, 2-thienyl-2-ethyl, 3-furyl-1-ethyl, 2-pyridyl-3-propyl, and the like groups.

The "acyl group" specifically includes formyl, acetyl, propionyl, butyryl, isobutyryl, valeryl, isovaleryl, pivaloyl, hexanoyl, acryloyl, propioloyl, metacryloyl, crotonoyl, benzoyl, naphthoyl, toluoyl, hydroatropoyl, atropoyl, cinnamoyl, furoyl, thenoyl, nicotinoyl, isonicotinoyl, glucoloyl, lactoyl, glyceroyl, tropoyl, benzyloyl, salicyloyl, anisoyl, vaniloyl, veratoroyl, piperoniroyl, protocatechoyl, galloyl, cyclopentanecarbonyl, cyclohexanecarbonyl, cycloheptanecarbonyl, 1-methyl cyclohexanecarbonyl, 1-isopentylcyclopentanecarbonyl, 1-isopentyl cyclohexanecarbonyl, tert-butoxycarbonyl, methoxycarbonyl, ethoxycarbonyl, 2-(1-isopentylcyclohexanecarbonylamino)phenylthiocarbonyl, and the like groups. Preferred are acetyl, tert-batoxycarbonyl, benzoyl, 1-methylcyclohexanecarbonyl, 1-isopentylcyclopentanecarbonyl, 1-isopentylcyclohexanecarbonyl, and 2-(1-isopentylcydohexanecarbonylamino)phenylthiocarbonyl.

The term "substituted or unsubstituted" of the "substituted or unsubstituted C₃₋₁₀ cycloalkyl group," the "substituted or unsubstituted C₅₋₈ cycloalkenyl group," and the "substituted or unsubstituted C₃₋₁₀ cycloalkyl C₁₋₁₀ alkyl group" described for R, R₁, and the like means that the group may be substituted with 1-4 substituents which may be the same or different and any position may be arbitrarily substituted without any limitation. Specific examples of these groups are the above-described straight chain or branched C₁₋₁₀ alkyl group; the above-described straight chain or branched C₂₋₁₀ alkenyl group; the above-described C₃₋₁₀ cycloalkyl group; the above-described C₁₋₁₀ cycloalkenyl group; the above-described C₃₋₁₀ cycloalkyl C₁₋₁₀ alkyl group; the above-described aryl group; an amino group; a C₁₋₄ lower alkylamino group such as methylamino, ethylamino, or the like groups; an acylamino group such as acetylamino, propionylamino, benzylamino, or the like groups; an oxo group; the above-described aralkyl group; the above-described arylalkenyl group, and the like.

The above substituents are recommended as substituents for R. Among these, preferred for R₁ are the above-described straight chain or branched C₁₋₁₀ alkyl group, the above-described C₃₋₁₀ cycloalkyl group, the above-described C₅₋₈ cycloalkenyl group, the above-described aryl group, and the above-described amino group.

The term "substituted or unsubstituted" of the "substituted or unsubstituted aryl group," the "5- or 6-membered heterocyclic group containing 1-3 nitrogen, oxygen, or sulfur atoms," the "substituted or unsubstituted aralkyl group," the "substituted or unsubstituted arylalkenyl group," the "substituted or unsubstituted arylthio group," and the "substituted or unsubstituted 5- or 6-membered heteroarylalkyl group" described with respect to R, R₁, and the like means that the groups may be substituted with 1-4, preferably 1-3, substituents which may be the same or different and any position may be arbitrarily substituted without particular restriction. Examples of these groups include the above-described straight chain or branched C₁₋₁₀ alkyl group, preferably a straight chain or branched C₁₋₆ aralkyl group; the above-described straight chain or branched C₂₋₁₀ alkenyl group, preferably a straight chain or branched C₂₋₆ alkenyl group; the above-described halogen atom; a nitro group; the above-described amino group that may be substituted with the above-described C₁₋₄ lower alkyl group or the above-described acyl group; a hydroxyl group; the above-described C₁₋₄ lower alkoxy group; the above-described C₁₋₄ lower alkylthio group; the above-described halo-C₁₋₄ lower alkyl group; the above-described acyl group; an oxo group, and the like.

The above substituents are recommended as substituents mainly for R₁. Among these, preferred for R the above-described straight chain or branched C₁₋₆ alkyl group, the above-described halogen atom, and a nitro group.

The "substituted or unsubstituted" of the "substituted or unsubstituted straight chain or branched C₁₋₁₀ alkyl group" described for R₁ and the like means that the group may be substituted with 1-3 substituents which may be the same or different and any position may be arbitrarily substituted without particular restriction. Examples of these groups are the above-described C₁₋₄ lower alkoxy group; the above-described C₁₋₄ lower alkyl group; the above-described amino group that may be substituted with an acyl or hydroxyl group; the above-described lower C₁₋₄ alkylthio group; a carbamoyl group; a hydroxyl group; the above-described halogen atom; the above-described acyloxy group containing an acyl group; a carboxyl group; the above-described acyl group; the above-described aryloxy group containing an aryl group that may be substituted; and the like.

The "substituted or unsubstituted" of the "C₁₋₄ lower alkyl group" described with respect to R₂ and the like means that the group may be substituted with 1-3 substituents which may be the same or different and any position may be arbitrarily substituted without particular restriction. Examples of the group include the above-described C₁₋₄ lower alkoxy group; the above-described amino group that may be substituted with the above-described C₁₋₄ lower alkyl group or the above-described acyl group; the above-described C₁₋₄ lower alkylthio group; a carbamoyl group; a hydroxyl group; a carboxyl group; the above-described acyl group; the above-described heterocyclic group (particularly aromatic heterocyclic groups such as thienyl or non-aromatic heterocyclic group such as tetrahydrofuryl); and the like.

The term "substituted or unsubstituted" of the "substituted or unsubstituted amino group" and the "substituted or unsubstituted ureido group" described with respect to R₁ means that the groups may be substituted with one or more, preferably 1-2, substituents which may be the same or different and any position may be arbitrarily substituted without particular restriction. Examples of these groups are the above-described C₁₋₄ lower alkyl group; a hydroxyl group; the above-described acyl group; the above-described aryl group which may be substituted with the above-described C₁₋₄ lower alkoxy group; and the like.

More specifically, preferred as the "straight chain or branched C₁₋₁₀ alkyl group" for R are methyl, ethyl, isopropyl, butyl, isobutyl, tert-butyl, heptyl, 1-propylbutyl, and 1-isobutyl-3-methylbutyl.

The "straight chain or branched C₂₋₁₀ alkenyl group" referred to as R are preferably allyl, vinyl, isopropenyl, 1-methyl-2-propenyl, 2-methyl-2-propenyl, 1-methyl-1-butenyl, crotyl, 1,3-dimethyl-2-butenyl, 1-pentenyl, and 1-methyl-2-pentenyl.

The "halo-C₁₋₄ lower alkyl group" for R means a C₁₋₄ lower alkyl group, particularly preferably a methyl group, substituted with the above-described halogen atom, particularly preferably fluorine and chlorine, with being a trifluoromethyl group preferred.

The "substituted or unsubstituted C₃₋₁₀ cycloalkyl group" for R means a C₃₋₁₀ cycloalkyl group (particularly preferably cyclopropyl, cyclobutyl, cyclopentyl, cyclohexyl, cycloheptyl, octahydroindenyl, decahydronaphthyl, adamantyl, and bicyclo[2.2.1]heptyl) that may be substituted with 1-4 substituents selected from the above-described straight chain or branched C₁₋₁₀ alkyl group (particularly preferably a C₁₋₈ alkyl group such as methyl, ethyl, propyl, isopropyl, butyl, isobutyl, tert-butyl, pentyl, isopentyl, 2,2-dimethylpropyl, 4-methylpentyl, 2-ethylbutyl, or the like), the above-described straight chain or branched C₂₋₁₀ alkenyl group (particularly preferably a C₂₋₁ alkenyl group such as 1-methylvinyl, 2-methylvinyl, 3-methyl-3-propenyl, or the like), the above-described C₃₋₁₀ cycloalkcyl group (particularly preferably a C₃₋₇ cycloalkyl group such as cyclopropyl, cyclopentyl, cyclohexyl, or the like), the above-described C₅₋₈ cycloalkenyl group (particularly preferably a C₅₋₆ cycloalkenyl group such as cyclopentenyl, cyclohexenyl, or the like), the above-described C₃₋₁₀ cycloalkyl C₁₋₁₀ alkyl group (particularly preferably a C₃₋₇Cycloalkyl C₁₋₄ alkyl group such as cyclopropylmethyl, 2-cyclopropylethyl, 2-cyclopentylethyl, cyclohexylmethyl, 2-cyclohexylethyl, or the like), the above-described aryl group (particularly preferably a phenyl group), an oxo group, the above described aralkyl group (particularly preferably a phenyl C₁₋₄ lower alkyl group such as benzyl, phenethyl, or the like), and the above-described arylalkenyl group (particularly preferably a 2-phenylvinyl group). Referable examples thereof include 2,2,3,3-tetramethylcycloprop), 1-isopentylcyclobutyl, 1-isopropylcyclopentyl, 1-isobutylcyclopentyl, 1-isopentylcyclopentyl, 1-cyclohexylmetthylcyclopenthyl, cyclohexyl, 1-methylcyclohexyl, 1-ethylcyclohexyl, 1-propylcyclohexyl, 1-isopropylcyclohexyl, 1-butylcyclohexyl, 1-isobutylcyclohexyl, 1-pentylcyclohexyl, 1-isopentylcyclohexyl, 1-(2,2-dimethylpropyl)cyclohexyl, 1-(4-methylpentyl)cyclohexyl, 1-(2-ethylbutyl) cyclohexyl, 4-tert-butyl-l-isopentylcyclohexyl, 1-cyclopropylcyclohexyl, 1-bicyclohexyl, 1-phenylcyclohexyl, 1-cyclopropylmethylcyclohexyl, 1-cyclohexylmethylcyclohexyl, 1-(2-cyclopropylethyl) cyclohexyl, 1-(2-cyclopentylethyl)cyclohexyl, 1-(2-cyclohexylethyl)cyclohexyl, 4-methylcyclohexyl, 4-propylcyclohexyl, 4-isopropylcyclohexyl, 4-tert-butylcyclohexyl, 4-pentylcyecyclohexyl, 4-bicyclohexyl, 1-isopentylcycloheptyl, 3a-octahydroindenyl, 4a-decahydronaphthyl, 1-adamantyl, and 7,7-dimethyl-1-(2-oxo)-bicyclo[2.2.1]heptyl. The site of substitution is not specifically limited, but particularly preferably at position 1. Any substitution group as described above may be used, but the straight chain or branched C₁₋₁₀ alkyl group is particularly preferred.

A particularly preferable example of the substituted C₃₋₁₀ cycloalkyl group is a 1-substituted-C₃₋₁₀ cycloalkyl group. The "1-substituted-C₃₋₁₀ cycloalkyl group" means a cycloalkyl group (for example, cyclopropyl, cyclobutyl, cyclopentyl, cyclohexyl, and cycloheptyl, preferably a C₅₋₇ cycloalkyl group, particularly preferably a cyclohexyl group) that is substituted at position 1 with substituents selected from the above-described straight chain or branched C₁₋₁₀ alkyl group (particularly preferably a C₁₋₈ alkyl group such as methyl, ethyl, propyl, isopropyl, butyl, isobutyl, pentyl, isopentyl, 2,2-dimethylpropyl, 4-methylpentyl, or 2-ethylbutyl), the above-described straight chain or branched C₂₋₁₀ alkenyl group (particularly preferably a C₂₋₈ alkenyl group such as 1-methylvinyl, 2-mothylvinyl, or 3-methyl-3-propenyl, the above-described C₃₋₁₀ cycloalkyl (particularly preferably a C₃₋₇ cycloalkyl group such as cyclopropyl, cyclopentyl, or cyclohexyl), the above-described C₅₋₈ cycloalkenyl group (particularly preferably a C₅₋₆ cycloalkenyl group such as cyclopentenyl or cyclohexenyl), the above-described C₃₋₁₀ cycloalkyl C₁₋₁₀ alkyl group (particularly preferably a C₃₋₇ cycloalkyl C₁₋₄ lower alkyl group such as cyclopropylmethyl, 2-cyclopropylethyl, 2-cyclopentylethyl, cyclohexylmethyl, or 2-cyclohexylethyl), the above-described aryl group (particularly preferably a phenyl group), the above-described aralkyl group (particularly preferably a phenyl C₁₋₄ lower alkyl group such benzyl and phenethyl), and an arylalkenyl group (particularly preferably 2-phenylvinyl). Preferable examples of the 1-substituted-C₃₋₁₀ cycloalkyl group include 1-isopentylcyclobutyl, 1-isopropylcyclopentyl, 1-isobutylcyclopentyl, 1-isopentyl cyclopentyl, 1-cyclohexylmethylcyclopentyl, 1-methylcyclohexyl, 1-ethylcyclohexyl, 1-propylcyclohexyl, 1-isopropylcyclohexyl, 1-butylcyclohexyl, 1-isobutylcyclohexyl, 1-pentylcyclohexyl, 1-isopentylcyclohexyl, 1-(2,2-dimethylpropyl)cyclohexyl, 1-(4-methylpentyl)cyclohexyl, 1-(2-ethylbutyl)cyclohexyl, 1-cyclopropylcyclohexyl, 1-bicyclohexyl, 1-phenylcyclobexyl, 1-cyclopropylmethylcyclohexyl), 1-cyclohexylmethylcyclohexyl, 1-(2-cyclopropylethyl)cyclohexyl, 1-(2-cyclopontylethyl)cyclohexyl, 1-(2-cyclohexylethyl)cyclohexyl, and 1-isopentylcycloheptyl. The straight chain or branched C₁₋₁₀ alkyl group is particularly preferable as a substituent at position 1.

The substituent for the "substituted or unsubstituted C₅₋₈ cycloalkenyl group" for R is the same as that for the above "substituted or unsubstituted C₃₋₁₀ cycloalkyl group." Specifically, it means a cycloalkenyl group (especially cyclopentenyl and cyclohexenyl) that may have 1-4 substituents selected from the above-described straight chain or branched C₁₋₁₀ alkyl group (particularly preferably a C₁₋₈ alkyl group such as methyl, ethyl, propyl, isopropyl, butyl, isobutyl, pentyl, isopentyl, 2,2-dimethylpropyl, 4-methylpentyl, or the like), the above-described straight chain or branched C₂₋₁₀ alkenyl group (particularly preferably a C₂₋₈ alkenyl group such as 1-methylvinyl, 2-methylvinyl, 3-methyl-3-propenyl, and the like), the above-described C₃₋₁₀ cycloalkyl group (particularly preferably a C₃₋₇ cycloalkyl group such as cyclopropyl, cyclopentyl, cyclohexyl, or the like), the above-described C₅₋₈ cycloalkenyl group (particularly preferably a C₅₋₆ cycloalkenyl group like cyclopentenyl, cyclohexenyl, or the like), the above-described C₃₋₁₀ cycloalkyl C₁₋₁₀ alkyl group (particularly preferably a C₃₋₇ cycloalkyl C₁₋₄ lower alkyl group such as cyclopropyl methyl, 2-cyclopropylethyl, 2-cyclopentylethyl, cyclohexylmethyl, 2-cyclohexylethyl, or the like), the above-described aryl.group (particularly preferably a phenyl group), an oxo group, the above-described aralkyl group (particularly preferably a phenyl C₁₋₄ lower alkyl group such as benzyl, phenethyl, or the like), and arylalkenyl group (particularly preferably 2-phenylvinyl). Preferable examples of the cycloalkenyl group includes 1-isopropyl-2-cyclopentenyl, 1-isopropyl-3-cyclopentenyl, 1-isobutyl-2-cyclopentenyl, 1-isobutyl-3-cyclopentenyl, 1-isopentyl-2-cyclopentenyl, 1-isopentyl-3-cyclopentenyl, 1-cyclohexylmethyl-2-cyclopentenyl, 1-cyclohexylmethyl-3-cyclopentenyl, 1-cyclohexenyl, 2-cyclohexenyl, 3-cyclohexenyl, 1-methyl-2-cyclohexenyl, 1-methyl-3-cyclohexenyl, ethyl-2-cyclohexenyl, 1-ethyl-3-cyclohexenyl, 1-propyl-2-cyclohexenyl, 1-propyl-3-cyclohexenyl, 1-isopropyl-2-cyclohexenyl, 1-isopropyl-3-cyclohexenyl, 1-butyl-2-cyclohexenyl, 1-butyl-3-cyclohexenyl, 1-isobutyl-2-cyclohexenyl, 1-isobutyl-3-cyclohexenyl, 1-pentyl-2-cyclohexenyl, 1-pentyl-3-cyclohexenyl, 1-isopentyl-2-cyclohexenyl, 1-isopentyl-3-cyclohexenyl, 1-(2,2-dimethytpropyl)-2-cyclohexenyl 1-(2,2-dimethylpropyl)-3-cyclohexenyl, 1-(4-methylpentyl)-2-cyclohexenyl, 1-(4-methylpentyl)-3-cyclohexenyl, 1-cyclopropyl-2-cyclohexenyl, 1-cyclopropyl-3-cyclohexenyl, 1-cyclohexyl-2-cyclohexenyl, 1-cyclohexenyl-3-cyclohexenyl, 1-phenyl-2-cyclohexenyl, 1-phenyl-3-cyclohexenyl, 1-cyclopropylmethyl-2-cyclohexenyl, 1-cyclo propylmethyt-3-cyclohexenyl, 1-cyclohexylmethyl-2-cyclohexenyl, 1-cyclohexylmethyl-3-cyclohexenyl, 1-(2-cyclopropylethyl)-2-cyclohexenyl, 1-(2-cyclopropylethyl)-3-cyclohexenyl, 1-(2-cyclopentylethyl)-2-cycloboxenyl, 1-(2-cyclopentylethyl)-3-cyclohexenyl, 1-(2-cyclohexylethyl)-2-cyclohexenyl, and 1-(2-cyclohexylethyl)-3-cyclohexenyl. There is no special restriction on the substitution position, but the particularly preferred position is position 1. Any one of the above substituents may be used, but the straight chain or branched C₁₋₁₀ alkyl group or the C₃₋₁₀ cycloalkyl C₁₋₄ alkyl group is particularly preferred.

A particularly preferable example of the substituted C₅₋₈ cycloalkenyl group is a 1-substituted-C₅₋₈ cycloalkenyl group. The "1-substituted-C₅₋₈ cycloalkenyl group" means a cycloalkenyl groups (particularly preferably a C₅₋₆ cycloalkenyl group such as cyclopentenyl, or cyclohexenyl) that is substituted at position 1 with substituents selected from the above-described straight chain or branched C₁₋₁₀ alkyl group (particularly preferably a C₁₋₈ alkyl group such as methyl, ethyl, propyl, isopropyl, butyl, isobutyl, pentyl, isopentyl, 2,2-dimethyl propyl, and 4-methylpentyl), the above-described straight chain or branched C₂₋₁₀ alkenyl group (particularly preferably a C₂₋₈ alkenyl group such as 1-methylvinyl, 2-methylvinyl, or 3-methyl-3-propenyl), the above-described C₃-₁₀ cycloalkyl group (particularly preferably a C₃-₇ cycloalkyl group such as cyclopropyl, cyclopentyl, or cyclohexyl), the above-described C₅-₈ cycloalkenyl group (particularly preferably a C₅-₆ cycloalkenyl group such as cyclopentenyl or cyclohexenyl), the above-described C₃-₁₀ cycloalkyl C₁₋₁₀ alkyl group (particularly preferably a C₃₋₇ cycloalkyl C₁-₄ lower alkyl group such as cyclopropylmethyl, 2-cyclopropylethyl, 2-cyclopentylethyl, cyclohexylmethyl, or 2-cyclohexylethyl), the above-described aryl group (particularly preferably a phenyl group), the abave-described aralkyl group (particularly preferably a phenyl C₁₋₄ lower alkyl group such as benzyl or phenethyl), and the above-described arylalkenyl group (particularly preferably a 2-phenylvinyl group). Preferable examples of the 1-substituted-C₅₋₈ cycloalkenyl group include 1-isopropyl-2-cyclopentenyl, 1-isopropyl-3-cyclopentenyl, 1-isobutyl-2-cyclopeutenyl, 1-isobutyl-3-cyclopentenyl, 1-isopentyl-2-cyclopentenyl, 1-isopentyl-3-cyclopentenyl, 1-cyclohexylmethyl-2-cyclopentenyl, 1-cyclohexylmethyl-3-cyclopentenyl, 1-methyl-2-cyclohexenyl, 1-methyl-3-cyclohexenyl, 1-ethyl-2-cyclohexenyl, l-ethyl-3-cyclohexenyl, 1-propyl-2-cyclohexenyl, 1-propyl-3-cyclohexenyl, 1-isopropyl-2-cyclohexenyl, 1-isopropyl-3-cyclohexenyl, 1-butyl-2-cyclohexenyl, 1-butyl-3-cyclohexenyl, 1-isobutyl-2-cyclohexenyl, 1-isobutyl-3-cyclohexenyl, 1-pentyl-2-cyclohexenyl, 1-pentyl-3-cyclohexenyl, 1-isopentyl-2-cyclohexenyl, 1-isopentyl-3-cyclohexenyl, 1-(2,2-dimetlrylpropyl)-2-cyclohexenyl, 1-(2,2-dimethylpropyl)-3-cyclohexenyl, 1-(4-methylpentyl)-2-cyclohexenyl, 1-(4-methylpentyl)-3-cyclohexenyl, 1-cyclopropyl-2-cyclohexenyl, 1-cyclopropyl-3-cyclohexenyl, 1-cyc1ohexyl-2-cyclohexenyl, 1-cyclohexyl-3-cyclohexenyl, 1-phenyl-2-cyclohexenyl, 1-phenyl-3-cyclohexenyl, 1-cyclopropylmethyl-2-cyclohexenyl, 1-cyclopropylmethyl-3-cyclohexenyl, 1-cyclohexylmethyl-2-cyclohexenyl, 1-cyclohexltmethyl-3-cyclohexenyl, 1-(2-cyclopropylethyl)-2-cyclohexenyl, 1-(2-cyclopropylethyl)-3-cyclohexenyl, 1-(2-cyclopentylethyl) -2-cyclohexenyl, 1-(2-cyclopentylethyl)-3-cyclohexenyl, 1-(2-cyclohexylethyl)-2-cyclohexenyl, and 1-(2-cyclohexylethyl)-3-cyclohexenyl. The straight chain or branched C₁₋₁₀ alkyl group is particularly preferable as a substituent at position 1.

The "substituted or unsubstituted C₃₋₁₀ cycloalkyl C₁₋₁₀ alkyl group" for R means a C₃-₁₀ cycloalkyl C₁₋₁₀ alkyl group (particularly preferably cyclohexylmethyl, 1-cyclohexylethyl, 1-cyclohexyl-1-methylethyl, 1-cyclohexyl-2-methylpropyl, 1-cyclohexyl-3-methylbutyl, 1-cyclohexylhexyl, 1-cyclohexyl-4-methylpentyl, and 1-cyclohexylheptyl), C₁₋₁₀ alkyl group of which is straight chain or branched and which may have 1-4 substituents selected from the above-described C₃-₁₀ cycloalkyl group (particularly preferably a C₃₋₇ cycloalkyl group such as cyclopentyl or cyclohexyl), the above-described C₅₋₈ cycloalkenyl group (particularly preferably a C₅-₇ cycloalkenyl group such as cyclopentenyl or cyclohexenyl), and the above-described aryl group (particularly preferably a phenol group). There is no special restriction on the substitution position. The above-described substituents may be placed at the straight chain or branched C₁₋₁₀ alkyl moiety. Preferable examples of the C₃-₁₀ cycloalkyl C₁₋₁₀ alkyl group include cyclohexylmethyl, 1-cyclohexylethyl, cyclohexyloyclopetltylmethyl, dicyclohexylmethyl, 1-cyclohexyl-1-methylethyl, 1-cyclohexyl-2-methylpropyl, 1-cyclohexyl-3-methylbutyl, 1-cyclohexyl-4-methylpentyl, 1-cyclohexylhexyl, and 1-cyclohexylheptyl.

The "substituted or unsubstituted aryl group" for R means an aryl group (particularly preferably a phenyl group) that may have 1-4 substituents selected from the above-described straight chain or branched C₁-₆ alkyl group (particularly preferably a tert-butyl group), the above-described halogen atom (particularly preferably fluorine and chlorine), and a nitro group. Preferable examples of the aryl group are phenyl, 2-chlorophenyl, 4-nitrophenyl, and 3,5-di-tert-butylphenyl.

The "substituted or unsubstituted aralkyl" for R means an aralkyl group (particularly preferably benzyl, benzhydryl, and trityl) which may have substituents selected from the above-described halogen atom (particularly preferably fluorine and chlorine), a nitro group, and a hydroxy group, and in which the C₁₋₄ lower alkyl group is straight chain or branched. There is no special restriction on the position of substitution. The straight chain or branched C₁-₄ lower alkyl moiety may be substituted. Preferable examples of the aralkyl group are benzyl and trityl.

The "substituted or unsubstituted 5- or 6-membered heterocyclic group having 1-3 nitrogen, oxygen or sulfur atoms" for R means the above-described heterocyclic group that may have 1-4 substituent selected from the above-described straight chain or branched C₁₋₆ alkyl group (particularly preferably a tert-butyl group), the above-described halogen atom (particularly preferably fluorine and chlorine), and a nitro group. The heterocyclic group is preferably an aromatic heterocyclic group, particularly preferably furyl, thienyl, and pyridyl.

The "substituted or unsubstituted straight chain or branched C₁₋₁₀ alkyl group" for R₁ means a straight chain or branched C₁₋₁₀ alkyl group that may have a substituent selected from the above-described halogen atom (particularly preferably fluorine and chlorine), the above-described C₁₋₄ lower alkoxy group (particularly preferably a methoxy group), an amino group that may be substituted with the above-described C₁₋₄lower alkyl group (particularly preferably a methyl group), the above-described acyl group (particularly preferably an acetyl, group), or a hydroxyl group, the above-described C₁₋₄ lower alkylthio group (particularly preferably a methylthio group), a carbamoyl group, a hydroxyl group, an acyloxy group having the above-described acyl group (particularly preferably an acetyloxy group), a carboxyl group, an acyl group (particularly preferably a methoxycarbonyl group), and an aryloxy group having the above-described substituted or unsubstituted aryl group (particularly preferably a phenoxy group and a 4-chlorophenoxy group). Preferable examples of the alkyl group include methyl, chloromethyl, ethyl, isopropyl, 1-methyl-2-pentyl, octyl, methoxymethyl, dimethylaminomethyl, acetylaminomethyl, 1-acetyl aminoethyl, 1-acetylamino-2-methylpropyl, 1-acetylamino-3-methylbutyl, 1-acetylamino-3-methylthiopropyl, 1-acetylamino-3-carbamoylpropyl, 1-hydroxy-1-methylethyl, 1-acetyloxy-1-methylethyl, 4-carboxybutyl, 2-methoxycarbonylethyl, phenoxymethyl, and 4-chlorophenoxymethyl.

The "C₁₋₄ lower alkoxy group" for R₁ is preferably a methoxy group and a tert-butoxy group.

The "C₁₋₄ lower alkylthio group" for R₁ is preferably a methylthio group.

The "substituted or unsubstituted amino group" for R₁ means an amino group that may have a substituent selected from the above-described C₁₋₄ lower alkyl group (particularly preferably ethyl, isopropyl, and tert-butyl), the above-described acyl group (particularly preferably acetyl and benzoyl), and the above-described aryl group (particularly preferably phenyl and 4-methoxyphenyl) that may be substituted with the above-described C₁₋₄ lower alkoxy group. Preferable examples of the amino group are ethylamino, isopropyl amino, tert-butylamino, phenylamino, and 4-methoxyphenylamino.

The "substituted or unsubstituted ureido group" for R₁ means a ureido group that may have a substituent selected from the above-described C₁₋₄ lower alkyl group (particularly preferably methyl and ethyl), the above-described acyl group (particularly preferably acetyl and benzoyl), and the above-described aryl group (particularly preferably phenyl and 4-methoxyphenyl) that may be substituted with the above-described C₁₋₄ lower alkoxy group, with an N,N'-diphenylureido group being preferred,

The "substituted or unsubstituted C₃₋₁₀ cycloalkyl group" for R₁ means a C₃₋₁₀ cycloalkyl group (particularly preferably cyclopropyl and cyclohexyl) that may have a substituent selected from the above-described straight chain or branched C₁₋₁₀ alkyl group (particularly preferably methyl, tert-butyl, and isopentyl), an amino group, an amino group (particularly preferably methyl amino, ethylamino, acetylamino, and benzylamino) that may be substituted with the above-described C₁₋₄ lower alkyl or acyl groups. Preferable examples the cycloalkyl group are cyclopropyl, cyclohexyl, 1-methylcyclohexyl, 1-isopentylcyclohexyl, 1-aminocyclohexyl, 1-acetylaminocyclohexyl, and 4-tert-butyicyaohexyl.

The "substituted or unsubstituted C₃₋₁₀ cycloalkyl C₁₋₁₀ alkyl group" for R₁ means a C₃₋₁₀ cycloalkyl C₁₋₁₀ alkyl group which may have a substituent selected from the above-described C₃₋₁₀ cycloalkyl group (particularly preferably cyclopentyl and cyclohexyl), the above-described C₃₋₈ cycloalkenyl group (particularly preferably cyclopentenyl and cyclohexenyl), and the above-described aryl group (particularly preferably a phenyl group) and in which the C₁₋₁₀ alkyl moiety is straight chain or branched. There is no special restriction on the position of substitution. The straight chain or branched C₁₋₁₀ alkyl moiety may be substituted. A cyclohexylmethyl group is preferred as the C₃₋₁₀ cycloalkyl C₁₋₁₀ alkyl group,

The "substituted or unsubstituted aryl group" for R₁ means an aryl group (particularly preferably phenyl and naphthyl) that may have a substituent selected from the above-described straight chain or branched C₁₋₆ alkyl group (particularly preferably methyl and tert-butyl group), the above-described halogen atom (particularly preferably fluorine and chlorine), a nitro group, a hydroxy group, the above-described C₁₋₄ lower alkoxy group (particularly preferably a methoxy group), and the above-described acyl group (particularly preferably a 2-(1-isopentylcyclohexanecarbonylamino)phenylthiocarbonyl group). Preferable examples of the aryl group include phenyl, 1-naphthyl, 2-naphthyl, 2-chlorophenyl, 2,6-dichlorophenyl, 2,6-dimethylphenyl, 2-methoxyphenyl, 2-nitrophenyl, 4-nitrophenyl, 3,5-di-tert-butyl-4-hydroxyphenyl, and 4-[2-(1-isopentylcyclohexanecarbonylamino)phenylthiocarbonyl]phenyl.

The "substituted or unsubstituted aralkyl group" for R₁ means an aralkyl group (particularly preferably benzyl, phenethyl, 3-phenylpropyl, naphthylmethyl, and biphenylmethyl) that may have a substituent selected from the above-described halogen atom (particularly preferably fluorine and chlorine), a nitro group, an amino group (particularly preferably amino, acetylamino, pivaloylamino, 1-methylcyclohexanecarbonylamino, tert-butoxycarbonylamino, and benzoylamino) that may be substituted with the above-described C₁₋₄ lower alkyl group or the above-described acyl group, and a hydroxyl group, and in which the C₁₋₄ lower alkyl group are straight chain or branched. There is no special restriction on the position of substitution. The straight chain or branched C₁₋₄ lower alkyl moiety may be substituted. Preferable examples of the aralkyl group include benzyl, phenethyl, 3-phenylpropyl, 2-naphthylmethyl, 4-biphenylmethyl, benzhydryl, 2-chlorophenylmethyl, 3-chlorophenylmethyl, 4-chlorophenylmethyl, 2-nitrophenylmethyl, 4-nitrophenylmethyl, 2-pivaloylaminophenylmethyl, 2-(1-methylcyclohexanecarbonylamino)phenylmethyl, 2-tert-butoxy-carbonylaminophenylmethyl, 3-acetylaminophenylmethyl, 3-(1-methylcyclohexanecarbonylamino)phenylmethyl, α-aminobenzyl, α-acetylaminobenzyl, α-(1-methylcyclohexanecarbonylamino)benzyl, α-benzoylaminobenzyl, α-aminophenethyl, α-acetylaminophenethyl, and 1-acetylamino-2-(4-hydorxyphenyl) ethyl.

The "substituted or unsubstituted arylalkenyl group" for R₁ means an arylalkenyl group (particularly phenylvinyl) that may have a substituent selected from the above-described straight chain or branched C₁₋₆ lower alkyl group (particularly preferably methyl and tert-butyl), the above-described halogen atom (particularly preferably fluorine and chlorine), a nitro group, and a hydroxyl group, with a 2-phenylvinyl group being preferred.

The "substituted or unsubstituted arylthio group" for R₁ means an arylthio group (particularly preferably a phenylthio group) that may have a substituent selected from the above-described halogen atom (particularly preferably fluorine and chlorine), a nitro group, and an amino group that may be substituted with the above-described C₂₋₄ lower alkyl group or the above-described acyl group (particularly preferably amino, acetylamino, pivaloylamino, 1-methylcyclohexanecarbonylamino, and benzoylamino), a hydroxyl group, and the above-described halo-C₁₋₄ lower alkyl group (particularly preferably a trifluoromethyl group). Preferably examples of the arylthio group include phenylthio, 2-pivaloylaminophenylthio, 2-(1-methylcyclohexanecarbonylamino)phenylthio, and 2-(1-methyl cyclohexanecarbonylamino-4-trifluoromethyl)phenylthio.

The "substituted or unsubstituted 5- or 6-membered heterocyclic ring groups with 1-3 nitrogen, oxygen, or sulfur atoms" for R₁ means heterocyclic ring groups (particularly preferably an aromatic heterocyclic group such as pyridyl or a non-aromatic heterocyclic group such as piperidyl or pyrrolidinyl) that may have substituents selected from the above-described straight chain or branched C₁₋₆ alkyl group (particularly preferably a methyl group), a halogen atom (particularly preferably fluorine and chlorine), the above-described acyl group (particularly preferably acetyl and benzoyl), and an oxo group. Preferable examples thereof are 3-pyridyl, 1-methyl-4-piperidyl, 1-acetyl-4-piperidyl, 5-oxo-2-pyrrolidinyl, 1-acetyl-2-pyrrolidinyl, and 1-benzoyl-2-pyrrolidinyl. A 4-piperidyl group such as 1-methyl-4-piperidyl or 1-acetyl-4-piperidyl group is particularly preferred.

The "substituted or unsubstituted 5- or 6-membered heteroarylalkyl group" for R₁ means the above-described heteroarylalkyl group (particularly preferably a 2-thenyl group) that may be substituted with the above-described straight chain or branched C₁₋₆ alkyl group (particularly preferably a methyl group) and the above-described halogen atom (particularly preferably fluorine and chlorine). A 2-thenyl group is preferred.

The "substituted or unsubstituted C₁₋₄ lower alkyl group" for R₂ means a C₁₋₄ lower alkyl group (particularly preferably a methyl group) that may have 1-3 substituents selected from the above-described C₁₄ lower alkoxy group (particularly preferably a methoxy group), an amino group that may be substituted with the above-described C₁₋₄ lower alkyl or acyl group (particularly preferably a dimethylamino group), the above-described C₁₋₄ lower alkylthio group (particularly preferably a methylthio group), a carbamoyl group, a hydroxyl group, a carboxyl group, the above-described acyl group (particularly preferably a methoxycarbonyl group), and the above-described heterocyclic group (particularly preferably an aromatic heterocyclic group such as thienyl or a non-aromatic heterocyclic group such as tetrahydrofuryl). A tetrahydrofurylmethyl group is preferred.

The "substituted or unsubstituted aryl group" for R₂ is the same as that for R₁. Preferable examples thereof are a phenyl group, a halogenated phenyl group, an acylamino-substituted phenyl group, and the like.

The "halogen atom" for X₁, X₂, X₃, and X₄ means a halogen atom including fluorine, chlorine, bromine, and the like, with fluorine and chlorine being preferred.

The "C₁₋₄ lower alkyl group" for X₁, X₂, X₃, and X₄ is preferably a methyl group.

The "halo-C₁₋₄ lower alkyl group" for X₁, X₂, X₃, and X₄ means a C₁₋₄ lower alkyl group (particularly preferably a methyl group) substituted with the above-described halogen atom (particularly preferably fluorine and chlorine). A trifluoromethyl group is preferred.

The "C₁₋₄ lower alkoxy group" for X₁, X₂, X₃, and X₄ is preferably a methoxy group.

The "acyl group" for X₁, X₂, X₃, and X₄ is preferably a benzoyl group.

The "aryl group" for X₁, X₂, X₃, and X₄ is preferably a phenyl group.

Preferably, the CETP inhibitor is a compound selected from the group consisting of N-(2-mercaptophenyl)-1-isopentylcyclohexanecarboxamide; N-(2-mercaptophenyl)-1-methylcyclohexanecarboxamide; N-(2-mercaptophenyl)-1-isopentylcyclopentanecarboxamide; N-(2-mercaptophenyl)-1-isopropylcyclohexanecarboxamide; N-(4,5-dichloro-2-mercaptophenyl)-1-isopentylcyclohexanecarboxamide; N-(4,5-dichloro-2-mercaptophenyl)-1-isopentylcyclopentanecarboxamide; N-(2-mercapto-5-methylphenyl)-1-isopentylcyclohexanecarboxamide; N-(2-mercapto-4-methylphenyl)-1-isopentylcyclohexanecarboxamide; S-[2-(1-isopentylcyclohexanecarbonylamino)phenyl] thioacetate; S-[2-(1-methylcyclohexanecarbonylamino)phenyl] 2,2-dimethylthiopropionate; S-[2-(1-isopentylcyclohexanecarbonylamino)phenyl] 2,2-dimethylthiopropionate; S-[2-(1-isopentylcyclohexanecarbonylamino)phenyl] 2-acetylamino-3-phenylthiopropionate; S-[2-(1-isopentylcyclohexanecarbonylamino)phenyl] 3-pyridinethiocarboxylate; S-[2-(1-isopentylcyclohexanecarbonylamino)phenyl] chlorathioacetate; S-[2-(1-isopentylcyclohexanecarbonylamino)phenyl] methoxythioacetatc; S-[2-(1-isopentylcyclohexanecarbonylamino)phenyl] thiapropionate; S-[2-(1-isopentylcyclohexanecarbonylamino)phenyl] phenoxythioacetate; S-[2-(1-isopentylcyclohexanecarbonylamino)phenyl] 2-methylthiopropionate; S-[2-(1-isopentylcyclohexanecarbanylamino)phenyl] 4-chlorophenoxythioacetate; S-[2-(1-isopentylcyclohexanecarbonylamino)phenyl] cyclopropanethiocarboxylate; S-[2-(1-isopentylcyclohexanecarbonylamino)phenyl] 2-acetylamino-4-carboxylthiobutyrate; S-[2-(1-isopentylcyclohexanecarbonylamino)phenyl] 2-hydroxy-2-methylthiopropionate; S-[2-(1-isopentylcyclopentanecarbonylamino)phenyl] 2,2-dimethylpropionate; 2-[2-(1-isopentylcyclopentanecarbonylamino)phenyl] thioacetate; S-[4,5-dichloro-2-(1 isopentylcyclohexanecarbonylamino)phenyl] 2,2-dimethylthiopropionate; S-[4,5-dichloro-2-(1-isopentylcyclopentanecarbonylamino)phenyl] 2,2-dimethylthiopropionate; S-[2-(1-isopentylcylohexanecarbonylamino)-4-trifluoromethylphenyl] 2,2-dimethylthiopropionate; O-methyl S-[2-(1-isopentylcydohexanecarbonylamino)phenyl] monothiocarbanate; S-[2-(1-methylcyclohexanecarbonylamino)phenyl] S-phenyl dithiocarbonate; S-[2-(1-isopentylcyclohexanecarbonylamino)phenyl] N-phenylthiocarbamate; S-[4,5-dichloro-2-(1-cyclopropylcyclohexanecarbonylamino)phenyl] 2,2-dimethyl-isopropionate; S-[4,5-dichloro-2-(1-pentylcyclohexanecarbonylamino)phenyl] 2,2-dimethylthiopropionate; S-[4,5-diclhoro-2-(1-cyclopropylmethylcyclohexanecarbonylamino)phenyl] 2,2-dimethylthiopropionate; S-[4,5-dichloro-2-(1-cyclohexylmethylcyclohexanecarbonylamino)phenyl] 2,2-dimethylthiopropionate; S-[4,5-dichloro-2-(1-isopropylcyclohexanecarbonylamino)phenyl] 2,2-dimethylthiopropionate; S-[4,5-dichloro-2-(1-isopentylcycloheptanecarbonylamino)phenyl] 2,2-dimethylthiopropionate; S-[4,5-dichloro-2-(1-isopentylcyclobutanecarbonylamino)phenyl] 2,2-dimethylthiopropionate; S-[2-(1-isopentylcyclohexanecarbonylanmino)-4-nitrophenyl] 2,2-dimethylthiopropionate; S-[4-cyano-2-(1-isopentylcyclohexanecarbonylamino)phenyl] 2,2-dimethylthiopropionate; S-[4-chloro-2-(1-isopentylcyclohexanecarbonylamino)phenyl] 2,2 -dimethylthiopropionate; S-[5-chloro-2-(1-isopentylcyclohexanecarbonylanino)phenyl] 2,2-dimethylthiopropionate; S-[4-fluoro-2-(1-isopentylcyclohexanecarbonylamino)phenyl] 2,2-dimethyhhiopropionate; S-[4,5-difluoro-2-(1-isopentylcyclohexanecarbonylamino)phenyl] 2,2-dimethylthiopropionate; S-[5-fluoro-2-(1-isopentylcyclohexanecarbonylamino)phenyl] 2,2-dimethylthiopropionate; N-(2-mercaptophenyl)-1-ethylcyclohexanecarboxamide; N-(2-mercaptophenyl)-1-propylcyclohexanecarboxamide; N-(2-mercaptophenyl)-1-butylcyclohexanecarboxamide; N-(2-mercaptophenyl)-1-isobutylcyclohexanecarboxamide; S-[2-(1-isopentylcyclohexanecarbonylamino)phenyl] cyclohexanethiocarboxylate; S-[2-(1-isopentylcyclohexanecarbonylamino)phenyl] thiobenzoate; S-[2-(1-isopentylcyclohexanecarbonylamino)phenyl] 5-carboxythiopentanoate; S-[2-(1-isopentylcyclohexanecarbonylamino)-4-methylphenyl] thioacetate; N-(2-mercaptophenyl)-1-(2-ethylbutyl)cyclohexanecarboxamide; S-[2-([[1-(2-ethylbutyl)cyclohexyl]carbonyl]amino)phenyl] 2-methylpropanethioate; S-[2-(1-isobutylcyclohexanecarbonylamino]phenyl] 2-methylthiopropionate; S-[2-[1-(2-ethylbutyl)cyclohexanecarbonylamino]phenyl] 1-acetylpiperidine-4-thiocarboxylate; S-[2-[1-(2-ethylbutyl)cyclohexanecarbonylamino]phenyl] thioacetate; S-[2-[1-(2-ethylbutyl)cyclohexanecarbonylamino]phenyl] 2,2-dimethylthiopropionate; S-[2-[1-(2-ethylbutyl)cyclohexanecarbonylamino]phenyl] methoxythioacetate; S-[2-[1-(2-ethylbutyl)cyclohexanecarbonylamino]phenyl] 2-hydroxy- 2-methylpropionate; S-[2-[1-(2-ethylbutyl)cyclohexanecarbonylamino]phenyl] 4-chlorophenoxythioacetate; S-[2-(1-isobutylcyclohexanecarbonylamino)phenyl] 4-chlorophenoxythioacetate; and S-[2-(1-isobutylcyclohexanecarbonylamino)phenyl] 1-acetylpiperidine-4-thiocarboxylate, or a prodrug compound, a pharmaceutically acceptable salt, a hydrate, or a solvate thereof.

Most preferably, the CETP inhibitor is *S*-[2-([[1-(2-ethylbutyl)cyclohexyl]carbonyl]amino)phenyl] 2-methylpropanethioate (which also is known as propanethioic acid, 2-methyl-, S-[2-[[[1-(2-ethylbutyl)cyclohexyl]carbonyl]amino]phenyl]ester; S-[1-[1-(2-ethylbutyl)cyclohexanecarbonylamino]phenyl] 2-methylthiopropionate, orJTT-705) (herein referred to as Compound I). Compound I has the following structural formula:

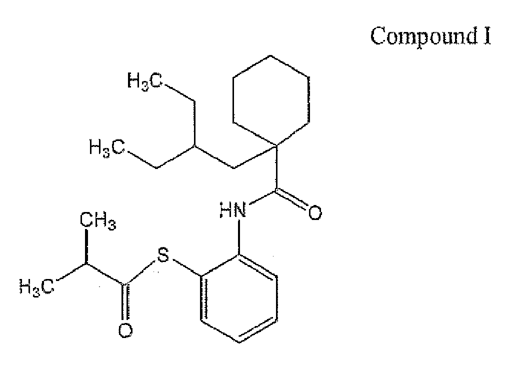

*S-*[2-([[1-(2-ethylbutyl)cyclohexyl]carbonyl]amino)phenyl]2-methylpropanethioate has been shown to be an inhibitor ofCETP activity in humans (de Grooth et al., Circulation, 105, 2159-2165 (2002)) and rabbits (Shinkai et al., J. Med. Chem., 43, 3566-3572 (2000); Kobayashi et al., Atherosclerosis, 162, 131-135 (2002); and Okamoto et al., Nature, 406(13), 203-207 (2000)), *S*-[2-([[1-(2-ethylbutyl)cyclohexyl]carbonyl]amino)phenyl] 2-methylpropanethioate has been shown to increase plasma HDL cholesterol in humans (de Grooth et al., *supra)* and in rabbits (Shinkai et al., *supra;* Kobayashi et al., *supra*; Okamoto et al., *supra).* Moreover, *S-*[2-([[1-(2-ethylbutyl)cyclohexyl]carbonyl]amino)phenyl] 2-methylpropanethioate has been shown to decrease LDL cholesterol in humans (de Grooth et al., *supra)* and rabbits (Okamoto et al, *supra).* Additionally, *S*-[2-([[1-(2-ethylbutyl)cyclohexyl]carbonyl]amino)phenyl] 2-methylpropanethioate inhibits the progression of atherosclerosis in rabbits (Okamoto et al *supra). S*-[2-([[1-(2-ethylbutyl)cyclohexyl]carbonyl]amino)phenyl] 2-methylpropanethioate, as well as methods of making and using the compound, are described in U.S. Patent 6,426,365.

While not wishing to be bound by any particular theory, it is hypothesized that within the body of a patient, Compound I is hydrolyzed in plasma, the liver, and/or the small intestine to form *S*-[2([[1-(2-ethylbutyl)cyclohexyl]carbonyl]amino)phenyl] thiol (herein referred to as Compound II). It is known that low molecular weight thiol components (i.e., R-SH), such as cysteine and glutathione, and high molecular weight thiol components (i.e., Prot-SH), such as peptides and proteins (e.g., enzymes and cell membranes), exist in the body as mixed disulfides containing an oxidized disulfide bond (S-S bond) between or within the molecule (see, e.g., Shimade et al., J. Chromatagr. B, 659, 227 (1994)). Therefore, it is hypothesized that within the body of a patient, Compound II is conjugated with low or high molecular weight thiols to yield mixed disulfides or to yield dimers of Compound II. Since these forms are in an oxidation-reduction equilibrium with each other via Compound II, all of these forms, as well as Compound II, are collectively, but not exclusively, considered and referred to hereafter as the active form of Compound I. The following scheme depicts the above-described hypothesis.

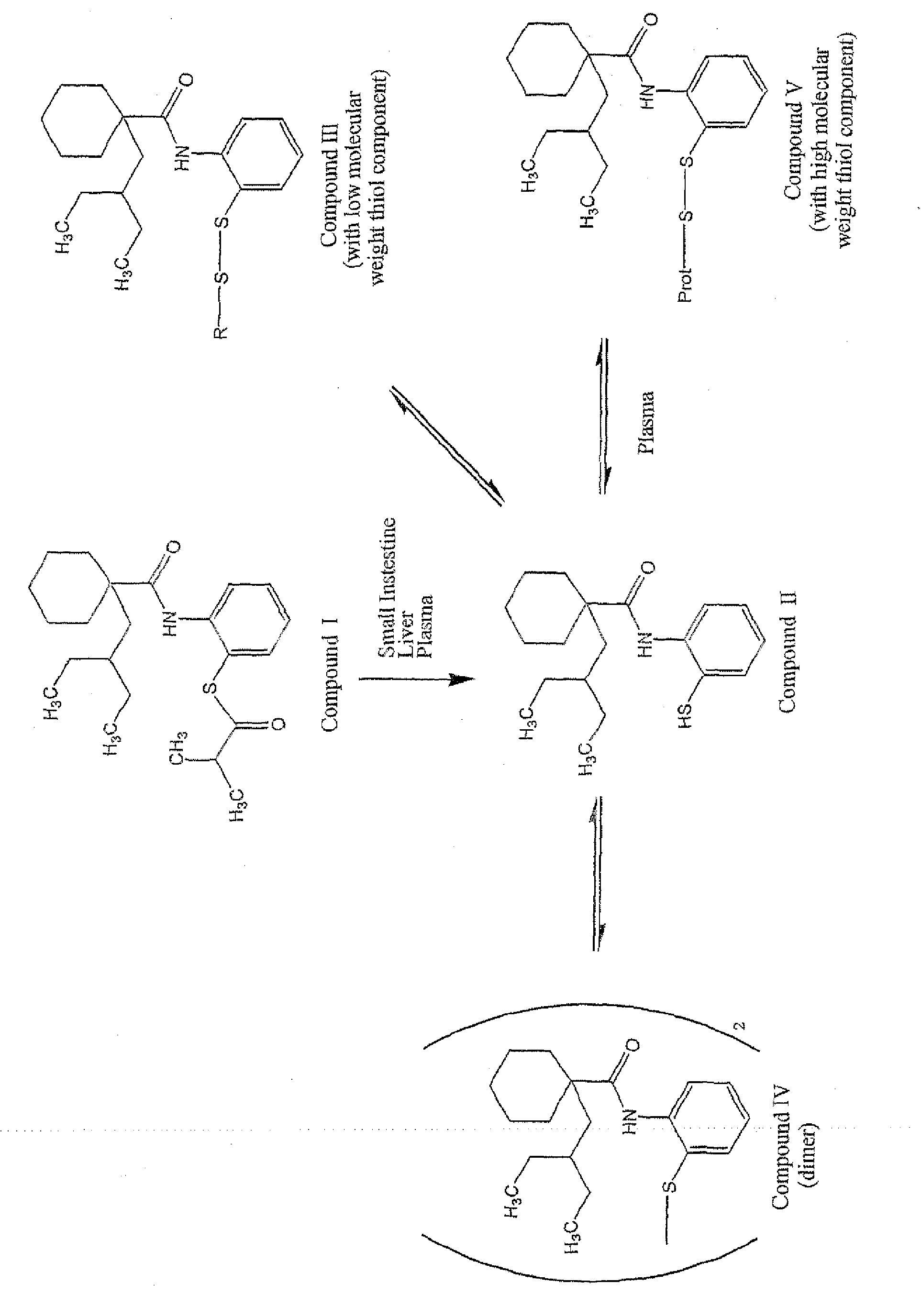

While the administration of Compound I is a particularly preferred embodiment of the invention, the invention also contemplates the administration of other compounds that will yield the active form of Compound I, i.e., other prodrugs of the active form of Compound 1. Such prodrugs, for example, can be compounds that have different mercapto-protecting groups, but that still result in the formation of the active form of Compound I (e.g., Compound II) in the body of a patient (i.e., *in vivo*). The term "mercapto-protecting groups" refers to commonly used mercapto-protecting groups (e.g., as described in Wolman, The Chemistry of the Thiol Group, D. Patai, Ed., Wiley-Interscience, New York, 1974). Any organic residues that can be dissociated *in vivo* may be used without particular restriction. Examples of particularly suitable mercapto-protecting groups are described in U.S. Patent 6,426,365. The invention further contemplates the administration of Compound I' (wherein R' signifies an organic residue other than an isopropyl group) so as to yield the active form of Compound 1.

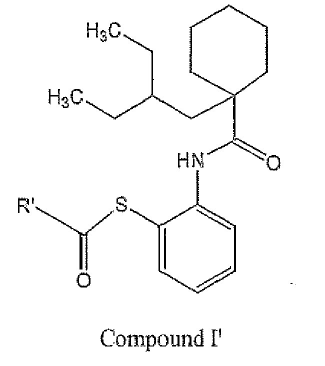

In addition, Compounds III, IV, and V (wherein R signifies an organic residue and Prot signifies a peptide or protein), which are believed to be in equilibrium with Compound II *in vivo*, similarly can be directly administered to the patient.

The CETP inhibitor (e.g., Compound I) can be in any suitable form (e.g., as a solid or a liquid, in crystalline or amorphous form, or any combination thereof). In a preferred embodiment, the CETP inhibitor is a solid in crystalline or amorphous form. The term "amorphous" signifies a non-crystalline state. The term "combination thereof" as applied to the amorphous or crystalline states of the CETP inhibitor refers to a mixture of amorphous and crystalline forms of the CETP inhibitor. A major portion of the CETP inhibitor can be in amorphous or crystalline form. As used herein, the term "a major portion" of the CETP inhibitor means more than 50% of the CETP inhibitor in the composition. For example, a major portion of the CETP inhibitor in the composition can be in crystalline form. Alternatively, the CETP inhibitor in the composition can be "substantially amorphous" (i.e., the amount of the CETP inhibitor in crystalline form does not exceed about 10%) or "substantially crystalline" (i.e., the amount of the CETP inhibitor in amorphous form does not exceed about 10%). Preferably, the CETP inhibitors is at least about 50% (e.g., at least about 55%, at least about 60%, at least about 65%, at least about 70%, at least about 75%, at least about 80%, at least about 85%, at least about 90%, at least about 95%, or actually about 100%) crystalline. The amount of crystalline CETP inhibitor can be measured by powder X-ray diffraction. Scanning Electron Microscope (SEM) analysis, differential scanning calorimetry (DSC), or any other standard quantitative measurement.

A substantial number of CETP inhibitors have a low aqueous solubility, a low bioavailability, and/or a slow rate of absorption such that it is desirable to increase their concentration in an aqueous environment of use. As used herein, the term "bioavailability" generally means the rate and extent to which the active ingredient, or active form thereof, is absorbed from a drug product and becomes available at the site of action. See U.S. Code of Federal Regulations, Title 21, Part 320.1 (2001 ed.). For oral dosage forms, bioavailability relates to the processes by which the active ingredient is released from the oral dosage form, e.g., a tablet, converted to the active form (if the active ingredient is not already the active form), and moved to the site of action, e.g., absorbed into the systemic circulation.

Oral delivery of many CETP inhibitors is often difficult because the aqueous solubility of CETP inhibitors is extremely low (i.e., the CETP inhibitor is substantially water-insoluble). The terms "extremely low aqueous solubility" and "substantially water-insoluble" signify that the CETP inhibitor has a maximum aqueous solubility of less than about 10 µg/mL (e.g., less than about 5 µg/mL, less than about 2 µg/mL, less than about 1 µg/mL, less than about 0.5 µg/mL, less than about 0.1 µg/mL, less than about 50 ng/mL, less than about 20 ng/mL, or less than about 10 ng/mL) at any physiologically relevant pH (e.g., pH 1-8) and at about 22°C. For example, the solubility of Compound I in water is less than about 0.0001 mg/mL. Such low solubilities are a direct consequence of the particular structural characteristics of the species that bind to CETP, and thus act as CETP inhibitors. This low solubility is primarily due to the hydrophobic nature of CETP inhibitors.

Thus, the hydrophobic and insoluble nature of CETP inhibitors poses a particular challenge for oral delivery. Achieving therapeutic drug levels in the blood by oral dosing of practical quantities of drug generality requires a large enhancement in drug concentrations in the gastrointestinal fluid and a resulting large enhancement in bioavailability. Additionally, the CETP inhibitors can have a very high dose-to-solubility ratio. Extremely low solubility often leads to poor or slow absorption of the drug from the fluid of the gastrointestinal tract, when the drug is dosed orally in a conventional manner. For extremely low solubility drugs, poor absorption generally becomes progressively more difficult as the dose (mass of drug given orally) increases.

The CETP inhibitors are characterized by a low melting point. The CETP inhibitors preferably have a melting point of about 150° C or less (e.g., about 140° C or less, about 130° C or less, about 120° C or less, about 110° C or less, about 100° C or less, about 90° C or less, about 80° C or less, or about 70° C or less. For example, Compound I has a melting point of about 63-65° C.

As a consequence of one or more of these properties, CETP inhibitors typically have very low absolute bioavailabilities. Specifically, the absolute bioavailibility of CETP inhibitors when dosed orally in their undispersed state is less than about 10% (e.g., less than about 9%, less than about 8%, less than about 7%, or less than about 6%) and more often less than about 5% (e.g., less than about 4%, less than about 3%, less than about 2%, or less than about 1 %).

To overcome the very low absolute bioavailabilities associated with CETP inhibitors, the invention provides a pharmaceutical composition comprising a CETP inhibitor and one or more water-insoluble concentration-enhancing additives. Advantageously, it has been found that inclusion of the water-insoluble concentration-enhancing additive dramatically improves the bioavailability of the CETP inhibitor.

The water-insoluble concentration-enhancing additive can be any suitable additive that enhances the bioavailability of the CETP inhibitor relative to the administration of the CETP inhibitor in the absence of the additive. The concentration-enhancing additive preferably is a polymer. By "water-insoluble" additive it is meant that the additive has a maximum aqueous solubility of less than about 10 µg/mL at any physiologically relevant pH (e.g., pH 1-8) and at about 22°C. By "concentration-enhancing additive(s)" it is meant that the additive (e.g., polymer) enhances the bioavailability of the CETP inhibitor relative to the administration of the CETP inhibitor in the absence of the concentration-enhancing additive(s). For example, the presence of an additive in the pharmaceutical composition preferably enhances the concentration of the CETP inhibitor (or active form thereof) in the aqueous environment of use (e.g., plasma, especially of a human) when compared to the administration of the CETP inhibitor in the absence of the additive. Thus, the additive can be considered a "concentration-enhancing additive" or, when such an additive is a polymer, a "concentration-enhancing polymer."

The pharmaceutical composition comprising a CETP inhibitor and a water-insoluble concentration-enhancing additive is capable of achieving specific maximum concentrations and areas under the concentration-time curve (AUC) from time zero up to the last quantifiable concentration (0-t_{z}) and/or from time zero to infinity (0-∞) of the active form of the CETP inhibitor (e.g., the active form of Compound I) in the environment of use (typically plasma, especially of a human), as discussed further in the description of the methods of use of the pharmaceutical compositions.

The amount of water-insoluble concentration-enhancing additive relative to the amount of CETP inhibitor present in the pharmaceutical composition depends on the CETP inhibitor and concentration-enhancing additive. The weight ratio of CETP inhibitor to additive can be from about 1:100 to about 10:1 (e.g., about 1:50, about 1:25, about 1:10, about 1:5, about 1:4; about 1:3, about 1:2, about 1:1, about 2:1, about 3:1, about 4:1, about 5:1, about 6:1, about 7:1, about 8:1, about 9:1, or ranges thereof). Preferably, the CETP inhibitor to additive weight ratio is about 2:1 1 to about 9:1, more preferably about 2:1 1 to about 4:1. The CETP inhibitor to additive ratio that yields optimum results varies from CETP inhibitor to CETP inhibitor and is best determined in *in vitro* dissolution tests and/or *in vivo* bioavailability tests.

An especially preferred water-insoluble concentration-enhancing additive is crospovidone (i.e., a synthetic homopolymer of cross-linked N-vinyl-2-pyrrolidone), Crospovidone can be present in the pharmaceutical composition in any suitable amount, desirably within the range of about 30% to about 100% (e.g., about 40%, about 50%, about 60%, about 70%, about 80%, about 90%, or ranges thereof) by weight of the CETP inhibitor (e.g., Compound I). In a preferred embodiment of the present invention, the weight ratio of CETP inhibitor to crospovidone can be from about 1:1 to about 3.3:1, more preferably about 2:1 1 to about 3:1. The amount of crospovidone in the pharmaceutical composition is important for disintegration and dissolution of the dosage form (e.g., tablet). For example, when the pharmaceutical composition comprises less than about 30% (e.g., less than about 25%, less than about 20%, less than about 15%, less than about 10%, or less than about 5°!°) of the CETP inhibitor by weight, the disintegration time of the tablet is delayed, and the resulting dissolved amount of the CETP inhibitor is decreased. The disintegration time and resulting dissolution amount are closely related to the absorbable amount of the CETP inhibitor in the gastrointestinal tract, which affects the efficacy level of the pharmaceutical composition.

The pharmaceutical composition comprising a CETP inhibitor and a water-insoluble concentration-enhancing additive (e.g., crospovidone) also can comprises one or more pharmacologically acceptable additives, such as pharmaceutically acceptable carriers or excipients and optionally other therapeutic agents (e.g., hydroxy-methylglutaryl coenzyme A reductase inhibitors) and/or components. For example, the CETP inhibitor can be used together with known pharmacologically acceptable carriers, excipients, diluents, extenders, disintegrants, stabilizers, preservatives, buffers, emulsifiers, aromatics, colorants, sweeteners, viscosity increasing agents, flavor improving agents, solubilizers, and other additives. These additives must be acceptable in the sense of being compatible with the other ingredients and not deleterious to the recipient thereof Examples of additives for oral administration include cornstarch, lactose, magnesium stearate, talc, microcrystalline cellulose, stearic acid, povidone, dibasic calcium phosphate, sodium starch glycolate, hydroxypropyl cellulose (e.g., low substituted hydroxypropyl cellulose), hydroxypropylmethyl cellulose (e.g., hydroxypropylmethyl cellulose 2910), and sodium lauryl sulfate.

The pharmaceutical composition desirably is in the form of a mixture, preferably a solid mixture, which is prepared by mechanically mixing the CETP inhibitor and water-insoluble concentration-enhancing additive, as well as additional pharmacologically acceptable additives. The composition is preferably substantially homogeneous so that the CETP inhibitor is dispersed as homogeneously as possible throughout the composition. As used herein, "substantially homogeneous" means that the fraction of CETP inhibitor that is present in relatively pure domains within the composition is relatively small, for example, less than about 20%, less than about 15%, less that about 10%, or less than about 5% of the total amount of CETP inhibitor.

The preferred solid composition may have one or multiple glass transition temperatures (T_{gs}). In one embodiment, the solid composition has a single glass transition temperature, which demonstrates that the composition is substantially homogeneous. T_{g} as used herein is the characteristic temperature where a glassy material, upon gradual heating, undergoes a relatively rapid (e.g., about 10 to about 100 seconds) physical change from a glass state to a rubber state. The Tg of a material can be measured by several techniques, including by a dynamic mechanical analyzer (DMA), a dilatometer, dielectric analyzer, and by a differential scanning calorimeter (DSC). The exact values measured by each technique can vary somewhat but usually fall within 10°C to 30°C of each other. Regardless of the technique used, when a composition exhibits a single T_{g}, this indicates that the composition is substantially homogenous.

The pharmaceutical composition can be prepared by any suitable method, such as those methods well known in the art of pharmacy, for example, methods such as those described in Gennaro et al., Remington's Pharmaceutical Sciences (18th ed., Mack Publishing Co., 1990), especially Part 8: Pharmaceutical Preparations and their Manufacture. Such methods include the step of bringing into association the CETP inhibitor with the other components of the pharmaceutical composition.

The pharmaceutical composition comprising the CETP inhibitor and water-insoluble concentration-enhancing additive can be made according to any suitable process. Preferably, the manufacturing process includes mechanical processes such as milling and extrusion. Alternatively, the pharmaceutical composition can be prepared by melt processes, such as high temperature fusion, solvent modified fusion, and melt-congeal processes; or solvent processes, such as non-solvent precipitation, spray coating, and spray-drying.

The pharmaceutical composition can provide controlled, slow release, or sustained release of the CETP inhibitor over a predetermined period of time. The controlled, slow release, or sustained release of the therapeutic compound can provide for a concentration of the CETP inhibitor, or the active form thereof, to be maintained in the bloodstream of the patient for a longer period of time. Such a pharmaceutical composition includes coated tablets, pellets, and capsules. Alternatively, the pharmaceutical composition can be in the form of a dispersion of the therapeutic compound in a medium that is insoluble in physiologic fluids or where the release of the therapeutic compound follows degradation of the pharmaceutical composition due to mechanical, chemical, or enzymatic activity.

The pharmaceutical composition can be, for example, in the form of a pill, capsule, or tablet, each containing a predetermined amount of the CETP inhibitor and preferably coated for ease of swallowing, in the form of a powder or granules. Preferably, the pharmaceutical composition is in the form of a tablet comprising the CETP inhibitor and the components of the tablet utilized and described in the Examples herein. For oral administration, fine powders or granules may contain diluting, dispersing, and or surface active agents and may be present, for example, in capsules or sachets in the dry state, or in tablets wherein binders and lubricants may be included. Components such as sweeteners, flavoring agents, preservatives (e.g., antimicrobial preservatives), suspending agents, thickening agents, and/or emulsifying agents also may be present in the pharmaceutical composition. A component of the formulation may serve more than one function.

Oral delivery methods are often limited by chemical and physical barriers imposed by the body, such as the varying pH in the gastrointestinal tract, exposure to enzymes, and the impermeability of the gastrointestinal membranes. The oral administration of the pharmaceutical composition may also include the co-administration of adjuvants. For example, nonionic surfactants such as polyoxyethylene oleyl ether and n-hexadecyl polyethylene ether can be administered with or incorporated into the pharmaceutical composition to artificially increase the permeability of the intestinal walls. Enzymatic inhibitors also can be administered with or incorporated into the pharmaceutical composition.

The pharmaceutical composition can be administered in any suitable manner. Preferably, the composition is administered with food. The term "with food" is defined to mean, in general, the condition of having consumed food during the period between from about 1 hour prior to the administration of the pharmaceutical composition comprising the CETP inhibitor to about 2 hours after the administration of the composition. Preferably, the food is a solid food with sufficient bulk and fat content that it is not rapidly dissolved and absorbed in the stomach. More preferably, the food is a meal, such as breakfast, lunch, or dinner.

Advantageously, the pharmaceutical composition is administered any time of day with food. The food can be consumed at any time during the period between from about 1 hour prior to the administration of the composition to about 2 hours after the administration of the composition. For example, the food can be consumed within the time period of about 1 hour, about 45 minutes, about 30 minutes, about 15 minutes, about 10 minutes, or about 5 minutes prior to the administration of the composition. Similarly, the food can be consumed within the time period of about 5 minutes, about 10 minutes, about 15 minutes, about 30 minutes, about 45 minutes, about 1 hour, about 1.25 hours, about 1.5 hours, about 1.75 hours, or about 2 hours after the administration of the composition. More preferably, the administration of the composition to the patient is immediately after the consumption of food (e.g. within about 1 minute after food consumption) up to about 1 hour after food consumption. Ideally, the pharmaceutical composition comprising the CETP inhibitor is administered at substantially the same time as the consumption of the food. The administration of the pharmaceutical composition with food can increase the bioabailability of the CETP inhibitor in the aqueous environment of use.

The terms "without food" or "fasted" are defined to mean the condition of not having consumed food within the time period of about 1 hour prior to the administration of the composition to about 2 hours after the administration of the composition.

The pharmaceutical composition can be used to treat or prevent a cardiovascular disorder, including, but not limited to, atherosclerosis, peripheral vascular disease, dyslipidemia (e.g., hyperlipidimia), hyperbetalipoproteinemia, hypoalphalipoproteinemia, hypercholesterolemia, hypertriglyceridemia, familial-hypercholesterolemia, angina, ischemia, cardiac ischemia, stroke, myocardial infarction, reperfusion injury, angioplastic restenosis, hypertension, cardiovascular disease, coronary heart disease, coronary artery disease, hyperlipidoproteinemia, vascular complications of diabetes, obesity or endotoxemia in a mammal, especially a human (i.e., a male or female human).

Accordingly, the invention provides a method for the treatment or prophylaxis of a cardiovascular disorder in a mammal, which method comprises administering to a mammal (preferably a mammal in need thereof) a therapeutically effective amount of the pharmaceutical composition. The mammal preferably is a human (i.e., a-male or female human). The human can be of any race (e.g., Caucasian or Oriental). The cardiovascular disorder preferably is selected from the group consisting of atherosclerosis, peripheral vascular disease, dyslipidemia, hyperbetalipoproteinemia, hypoalphalipoproteinemia, hypercholesterolemia, hypertriglyceridemia, familial-hypercholesterolemia, angina, ischemia, cardiac ischemia, stroke, myocardial infarction, reperfusion injury, angioplastic restenosis, hypertension, and vascular complications of diabetes, obesity or endotoxemia in a mammal, More preferably, the cardiovascular disorder is selected from the group consisting of cardiovascular disease, coronary heart disease, coronary artery disease, hypoalphalipoproteinemia, hyperbetalipoproteinemia, hypcrcholesterolemia, hyperlipidemia, atherosclerosis, hypertension, hypertriglyceridemia, hyperlipidoproteinemia, peripheral vascular disease, angina, ischemia, and myocardial infarction.

The CETP inhibitor can be administered to the mammal at any suitable dosage (e.g., to achieve a therapeutically effective amount). For example, a suitable dose of a therapeutically effective amount of Compound I for administration to a patient will be between approximately 100 mg to about 1800 mg per day. A desirable dose is preferably about 300 mg to about 900 mg per day. A preferred dose is about 600 mg per day. The phannacokinetics parameters (e.g., area under the concentration-time curve, maximum concentration, and the like) will, of course, vary based on the dosage administered to the mammal (e.g., human). The pharmacokinetics parameters may also be influenced by additional factors, such as the mass of the mammal and genetic components. For example, as illustrated by Examples 1-4, the bioavailability of Compound I (as indicated by Cma_{x,} AUC_{0-tz}, and AUC_{0-∞}) is greater following administration to Oriental (especially Japanese) humans as compared to Caucasian humans.

If desired, the effective daily dose of the CETP inhibitor (e.g., Compound I) may be administered as two, three, four, five, six, or more sub-doses administered separately at appropriate intervals throughout the day, optionally, in unit dosage forms. Each such subdose contains a therapeutically effective amount of the CETP inhibitor (e.g., Compound I).

The pharmaceutical composition, when administered to a mammal, especially a human, desirably achieves certain pharmacokinetic effects as evaluated by the maximum observed plasma concentration of the active form of CETP inhibitor (e.g., the active form of Compound I) (Cₘₐₓ), the area under the plasma concentration-time curve (AUC) from time zero up to the last quantifiable concentration (0-t_{z}) and/or from time zero to infinity (0-∞) of the CETP inhibitor (e.g., the active form of Compound 1), and/or a decrease in CETP activity (as compared to CETP activity before administration of the pharmaceutical composition).

The pharmaceutical composition, at a daily dose of 300 mg, 600 mg, or 900 mg of the CETP inhibitor, particularly Compound I, administered with food, preferably achieves a Cₘₐₓ, AUC_{0-tz}, AUC_{0-∞}, and/or decrease in CETP activity in the environment of use (e.g., plasma, especially of a human), as set forth below:
Cₘₐₓ(300 mg daily dose): at least about 0.1 µg/mL (e.g., at least about 0.15 µg/mL, at least about 0.2 µg/mL, at least about 0.25 µg/mL, at least about 0.3 µg/mL, at least about 0.4 µg/mL, at least about 0.5 µg/mL, at least about 0.6 µg/mL, at least about 0.7 µg/mL, at least about 0.8 µg/mL, at least about 0.9 µg/mL, at least about 1 µg/mL, at least about 1.1 µg/mL, at least about 1.2 µg/mL, at least about 1.3 µg/mL, at least about 1.4 µg/mL, at least about 1.5 µg/mL, at least about 1.6 µg/mL, at least about 1.7 µg/mL, or at least about 1.8 µg/mL).
Cₘₐₓ (600 mg daily dose): at least about 0.35 µg/mL (e.g., at least about 0.4 µg/mL, at least about 0.5 µg/mL, at least about 0.6 µg/mL, at least about 0.7 µg/mL, at least about 0.8 µg/mL, at least about 0.9 µg/mL, at least about 1 µg/mL, at least about µg/mL, at least about 1.2 µg/mL, at least about 1.3 µg/mL, at least about 1.4 µg/mL, at least about 1.5 µg/mL, at least about 1.6 µg/mL, at least about 1.7 µg/mL, at least about 1.8 µg/mL, at least about 1.9 µg/mL, or at least about 2 µg/mL).
Cₘₐₓ (900 mg daily dose): at least about 0.8 µg/mL (e.g., at least about 0.9 µg/mL, at least about 1 µg/mL, at least about 1.1 µg/mL, at least about 1.2 µg/mL, at least about 1.3 µg/mL, at least about 1.4 µg/mL, at least about 1.5 µg/mL, at least about 1.6 µg/mL, at least about 1,7 µg/mL, at least about 1.8 µg/mL, at least about 1.9 µg/mL, at least about 2 µg/mL, at least about 2.1 µg/mL, at least about 2.2 µg/mL, at least about 2.3 µg/mL, at least about 2.4 µg/mL, or at least about 2.5 µg/mL).
AUC_{0-tz},(300 mg daily dose): at least about 0.5 µg·h/mL (e.g., at least about 1 µg·h/mL at least about 1.5 µg·h/mL, at least about 2 µg·h/mL, at least about 2.5 µg·h/mL, at least about 3 µg·h/mL, at least about 3.5 µg·h/mL, at least about 4 µg·h/mL, at least about 4.5 µg·h/mL, at least about 5 µg·h/mL, at least about 5.5 µg·h/mL, at least about 6 µg·h/mL, at least about 6.5 µg·h/mL, at least about 7 µg·h/mL, at least about 7.5 µg·h/mL, at least about 8 µg·h/mL, at least about 8.5 µg·h/mL, at least about 9 µg·h/mL, at least about 9.5 µg·h/mL, or at least about 10 µg·h/mL).
AUC_{0-tz}(600 mg daily dose): at least about 3.5 µg·h/mL (e.g., at least about 4 µg·h/mL, at least about 4.5 µg·h/mL, at least about 5 µg·h/mL, at least about 5.5 µg·h/mL, at least about 6 µg·h/mL, at least about 6.5 µg·h/mL, at least about 7 µg·h/mL, at least about 7.5 µg·h/mL, at least about 8 µg·h/mL, at least about 8.5 µg·h/mL, at least about 9 µg·h/mL, at least about 9.5 µg·h/mL, at least about 10 µg·h/mL, at least about 10.5 µg·h/mL, at least about 11 µg·h/mL, at least about 11.5 µg·h/mL, at least about 12 µg·h/mL, at least about 12.5 µg·h/mL, at least about 13 µg·h/mL, at least about 13.5 µg·h/mL, at least about 14 µg·h/mL, at least about 14.5 µg·h/mL, or at least about 15 µg·h/mL).
AUC(_{0-tz}) (900 mg daily dose): at least about 7.5 µg·h/mL, at least about 8 µg·h/mL at least about 8.5 µg·h/mL, at least about 9 µg·h/mL,at least about 9,5 µg·h/mL, at least about 10 µg·h/mL, at least about 10.5 µg·h/mL, at least about 11 µg·h/mL, at least about 11.5 µg·h/mL, at least about 12 µg·h/mL, at least about 12.5 µg·h/mL, at least about 13 µg·h/mL, at least about 13.5 µg·h/mL; at least about 14 µg·h/mL, at least about 14.5 µg·h/mL, at least about 15 µg·h/mL, at least about 15.5 µg·h/mL, at least about 16 µg·h/mL, at least about 16.5 µg·h/mL, at least about 17 µg·h/mL, at least about 17.5 µg·h/mL, at least about 18 µg·h/mL, at least about 18.5 µg·h/mL, at least about 19 µg·h/mL, at least about 19,5 µg·h/mL, or at least about 20 µg·h/mL).
AUC_{0-∞} (300 mg daily dose): at least about 0.5 µg·h/mL (e.g., at least about 1 µg·h/mL, at least about 1.5 µg·h/mL, at least about 2 µg·h/mL, at least about 2.5 µg·h/mL, at least about 3 µg·h/mL, at least about 3.5 µg·h/mL, at least about 4 µg·h/mL, at least about 4.5 µg·h/mL, at least about 5 µg·h/mL, at least about 5.5 µg·h/mL, at least about 6 µg·h/mL, at least about 6.5 µg·h/mL, at least about 7 µg·h/mL, at least about 7.5 µg·h/mL, at least about 8 µg·h/mL, at least about 8.5 µg·h/mL, at least about 9 µg·h/mL, at least about 9.5 µg·h/mL, or at least about 10 µg·h/mL).
AUC_{0-∞} (600 mg daily dose): at least about 3.5 µg·h/mL (e.g., at least about 4 µg·h/mL, at least about 4.5 µg·h/mL, at least about 5 µg·h/mL, at least about 5.5 µg·h/mL, at least about 6 µg·h/mL, at least about 6.5 µg·h/mL,at least about 7 µg·h/mL, at least about 7.5 µg·h/mL, at least about 8 µg·h/mL, at least about 8.5 µg·h/mL, at least about 9 µg·h/mL, at least about 9.5 µg·h/mL, at least about 10 µg·h/mL, at least about 10.5 µg·h/mL, at least about 11 µg·h/mL, at least about 11.5 µg·h/mL, at least about 12 µg·h/mL, at least about 12.5 µg·h/mL, at least about 13 µg·h/mL, at least about 13.5 µg·h/mL, at least about 14 µg·h/mL, at least about 14.5 µg·h/mL, or at least about 15 µg·h/mL).
AUC(_{0-∞}) (900 mg daily dose): at least about 7.5 µg·h/mL, at least about 8 µg·h/mL, at least about 8.5 µg·h/mL, at least about 9 µg·h/mL, at least about 9.5 µg·h/mL, at least about 10 µg·h/mL, at least about 10.5 µg·h/mL, at least about 11 µg·h/mL, at least about 11.5 µg·h/mL, at least about 12 µg·h/mL, at least about 12.5 µg·h/mL, at least about 13 µg·h/mL, at least about 13.5 µg·h/mL, at least about 14 µg·h/mL, at least about 14.5 µg·h/mL, at least about at least about 15 µg·h/mL, at least about 15.5 µg·h/mL, at least about 16 µg·h/mL, at least about 16.5 µg·h/mL, at least about 17 µg·h/mL, at least about 17.5 µg·h/mL, at least about 18 µg.h/mL, at least about 18.5 µg·h/mL, at least about 19 µg·h/mL, at least about 19.5 µg·h/mL, or at least about 20 µg·h/mL).

Decrease in CETP activity (300 mg daily dose): at least about 10% (e.g., at least about 15%, at least about 20%, at least about 25%, at least about 30%, at least about 35%, at least about 40%, at least about 45%, at least about 50%, at least about 55%, at least about 60%, at least about 65%, at least about 70%, or at least about 75% or more) relative to the CETP activity before administration of the pharmaceutical composition.

Decrease in GETP activity (600 mg daily dose): at least about 25% (e.g., at least about 30%, at least about 40%, at least about 45%, at least about 50%, at least about 55%, at least about 60%, at least about 65%, at least about 70%, at least about 75%, at least about 80%, or at least about 85% or more) relative to the CETP activity before administration of the pharmaceutical composition.

Decrease in CETP activity (900 mg daily dose): at least about 35% (e.g., at least about 40%, at least about 45%, at least about 50%, at least about 55%, at least about 60%, at least about 65%, at least about 70%, at least about 75%, at least about 80%, at least about 85%, at least about 90%, or at least about 95% or more) relative to the CETP activity before administration of the Pharmaceutical composition.

The pharmaceutical composition, at a daily dose of 300 mg, 600 mg, or 900 mg of the CETP inhibitor, particularly Compound I, administered without food, preferably achieves a Cₘₐₓ, AUC_{0-tz}, AUC_{0-∞}, and/or decrease in CETP activity as set forth below:
Cₘₐₓ(300 mg daily dose): at least about 0.05 µg/mL (e.g., at least about 0.1 µg/mL, at least about 0.15 µg/mL, at least about 0.2 µg/mL, at least about 0.25 µg/mL, at least about 0.3 µg/mL, at least about 0.4 µg/mL, at least about 0.5 µg/mL, at least about 0.6 µg/mL, at least about 0.7 µg/mL, at least about 0.8 µg/mL, at least about 0.9 µg/mL, at least about 1 µg/mL, at least about 1.1 µg/mL, at least about 1.2 µg/mL, at least about 1.3 µg/mL, at least about 1,4 µg/mL, or at least about 1.5 µg/mL).
Cₘₐₓ (600 mg daily dose): at least about 0.15 µg/mL (e.g., at least about 0.2 µg/mL, at least about 0.25 µg/mL, at least about 0.3 µg/mL, at least about 0.4 µg/mL. at least about 0.5 µg/mL, at least about 0.6 µg/mL, at least about 0.7 µg/mL, at least about 0.8 µg/mL, at least about 0.9 µg/mL, at least about 1 µg/mL, at least about 1.1 µg/mL, at least about 1.2 µg/mL, at least about 1.3 µg/mL, at least about 1.4 µg/mL, at least about 1.5 µg/mL, at least about 1.6 µg/mL, at least about 1.7 µg/mL, at least about 1.8 µg/mL, at least about 1.9 µg/mL, or at least about 2 µg/mL).
Cₘₐₓ (900 mg daily dose): at least about 0.35 µg/mL (e.g., at least about 0.4 µg/mL, at least about 0.5 µg/mL, at least about 0.6 µg/mL, at least about 0.7 µg/mL, at least about 0.8 µg/mL, at least about 0.9 µg/mL, at least about 1 µg/mL, at least about 1.1 µg/mL, at least about 1.2 µg/mL, at least about 1.3 µg/mL, at least about 1.4 µg/mL, at least about 1.5 µg/mL, at least about 1.6 µg/mL, at least about 1.7 µg/mL, at least about 1.8 µg/mL, at least about 1.9 µg/mL, or at least about 2 µg/mL).
AUC_{0-tz}(300 mg daily dose): at least about 0.1 µg·h/mL (e.g., at least about 0.2 µg·h/mL, at least about 0.3 µg·h/mL, at least about 0.4 µg·h/mL, at least about 0.5 µg·h/mL, at least about 0.6 µg·h/mL, at least about 0.7 µg·h/mL, at least about 0.8 µg·h/mL, at least about 0.9 µg·h/mL, at least about 1 µg·h/mL, at least about 1.5 µg·h/mL, at least about 2 µg·h/mL, at least about 2.5 µg·h/mL, at least about µg·h/mL, at least about 3.5 µg·h/mL, at least about 4 µg·h/mL, at least about 4.5 µg·h/mL, at least about 5 µg·h/mL, at least about 5.5 µg·h/mL, at least about µg·h/mL, at least about 6.5 µg·h/mL, at least about 7 µg·h/mL, or at least about 7.5 µg·h/mL).
AUC_{0-tz} (600 mg daily dose): at least about 1.5 µg·h/mL (e.g., at least about 2 µg·h/mL, at least about 2.5 µg·h/mL, at least about 3 µg·h/mL, at least about 3.5 µg·h/mL, at least about 4 µg·h/mL, at least about 4.5 µg·h/mL, at least about 5 µg·h/mL, at least about 5.5 µg·h/mL, at least about 6 µg·h/mL, at least about 6.5 µg·h/mL, at least about 7 µg·h/mL, at least about 7.5 µg·h/mL, at least about 8 µg·h/mL, at least about 8.5 µg·h/mL, at least about 9 µg·h/mL, at least about 9.5 µg·h/mL, or at least about 10 µg·h/mL).
AUC(_{0-tz}) (900 mg daily dose): at least about 5.5 µg·h/mL (e.g., at least about 6 µg·h/mL, at least about 6.5 µg·h/mL, at least about 7 µg·h/mL, at least about 7.5 µg·h/mL, at least about 8 µg·h/mL, at least about 8.5 µg·h/mL, at least about 9 µg·h/mL, at least about 9.5 µg·h/mL, at least about 10 µg·h/mL, at least about 10.5 µg·h/m, at least about 11 µg·h/mL, at least about 11.5 µg·h/mL, at least about 12 µg·h/mL, at least about 12.5 µg·h/mL, at least about 13 µg·h/mL, at least about 13.5 µg·h/mL, at least about 14 µg·h/mL, at least about 14.5 µg·h/mL, at least about at least about 15 µg·h/mL, at least about 15.5 µg·h/mL, at least about 16 µg·h/mL, at least about 16.5 µg·h/mL,at least about 17 µg·h/mL, or at least about 17.5 µg·h/mL).
AUC_{0-∞} (300 mg daily dose): at least about 0.1 µg·h/mL (e.g., at least about 0.2 µg·h/mL, at least about 0.3 µg·h/mL, at least about 0.4 µg·h/mL, at least about 0.5 µg·h/mL, at least about 0.6 µg·h/mL, at least about 0.7 µg·h/mL, at least about 0.8 µg·h/mL, at least about 0.9 µg·h/mL, at least about 1 µg·h/mL, at least about 1.5 µg·h/mL, at least about 2 µg·h/mL, at least about 2.5 µg·h/mL, at least about 3 µg·h/mL, at least about 3.5 µg·h/mL, at least about 4 µg·h/mL, at least about 9.5 µg·h/mL, at least about 5 µg·h/mL, at least about 5.5 µg·h/mL, at least about 6 µg·h/mL, at least about 6.5 µg·h/mL, at least about 7 µg·h/mL, or at least about 7.5 µg·h/mL).
AUC_{0-∞} (600 mg daily dose): at least about 1.5 µg·h/mL (e.g., at least about 2 µg·h/mL, at least about 2.5 µg·h/mL, at least about 3 µg·h/mL, at least about 3.5 µg·h/mL, at least about 4 µg·h/mL, at least about 4.5 µg·h/mL, at least about 5 µg·h/mL, at least about 5.5 µg·h/mL, at least about 6 µg·h/mL, at least about 6.5 µg·h/mL, at least about 7 µg·h/mL, at least about 7.5 µg·h/mL, at least about 8 µg·h/mL, at least about 8.5 µg·h/mL, at least about 9 µg·h/mL, at least about 9.5 µg·h/mL, or at least about 10 µg·h/mL).
AUC(_{0-∞}) (900 mg daily dose): at least about 5.5 µg·h/mL (e.g., at least about 6 µg·h/mL, at least about 6.5 µg·h/mL, at least about 7 µg·h/mL, at least about 7.5 µg·h/mL, at least about 8 µg·h/mL, at least about 8.5 µg·h/mL, at least about 9 µg·h/mL, at least about 9.5 µg·h/mL, at least about 10 µg·h/mL, at least about 10.5 µg·h/mL, at least about 11 µg·h/mL, at least about 11.5 µg·h/mL, at least about 12 µg·h/mL, at least about 12.5 µg·h/mL, at least about 13 µg·h/mL, at least about 13.5 µg·h/mL, at least about 14 µg·h/mL, at least about 14.5 µg·h/mL, at least about 15 µg·h/mL, at least about 15.5 µg·h/mL, at least about 16 µg·h/mL, at least about 16.5 µg·h/mL, at least about 17 µg·h/mL, or at least about 17.5 µg·h/mL).

Decrease in CETP activity (300 mg daily dose): at least about 2.5% (e.g., at least about 5%, at least about 7.5%, at least about 10%, at least about 15%, at least about 20%, at least about 25%, at least about 30%, at least about 35%, at least about 40%, at least about 45%, or at least about 50% or more) relative to the CETP activity before administration of the pharmaceutical composition.

Decrease in CETP activity (600 mg daily dose): at least about 5% (e.g., at least about 7.5%, at least about 10%, at least about 15%, at least about 20%, at least about 25%, at least about 30%, at least about 35%, at least about 40%, at least about 45%, at least about 50%, at least about 55%, or at least about 60% or more) relative to the CETP activity before administration of the pharmaceutical composition.

Decrease in CETP activity (900 mg daily dose): at least about 12.5% (e.g., at least about 15%, at least about 20%, at least about 25%, at least about 30%, at least about 35%, at least about 40%, at least about 45%, at least about 50%, at least about 55%, at least about 60%, at least about 65%, at least about 70%, or at least about 75% or more) relative to the CETP activity before administration of the pharmaceutical composition.

Furthermore, the pharmaceutical composition of the invention, when administered to a patient, desirably results in one or more (e,g., two or three) of the following conditions in the patient: (a) an inhibition of cholesteryl ester transfer protein (CETP) activity in the patient relative to pretreatment CETP activity (as described above), (b) an increase in high density lipoprotein cholesterol (HDL-C) level in the patient relative to pretreatment HDL-C level, and (c) a decrease in the ratio of total cholesterol to HDL-C level (TC/HDL-C) in the patient relative to pretreatment TC/HDL-C. The term "pretreatment" refers to the time prior (desirably immediately prior) to administration of the active compounds of the composition of the invention to the patient. The desired extent of changes in each of the foregoing conditions in the patient relative to pretreatment are recited below.

The HDL-C level is measured using standard techniques known in the art. Preferably, the HDL-C level following the administration (e.g. after 4 weeks of treatment) of 300 mg of the CETP inhibitor, particularly Compound I, is increased by about 10% or more relative to pretreatment HDL-C level (e.g., about 12.5% or more, about 15% or more, about 17.5% or more, about 20% or more, about 22.5% or more, about 25% or more, about 27.5% or more, about 30% or more, about 32.5% or more, about 35% or more, about 37.5% or more, about 40% or more, about 42.5% or more, about 45% or more, about 47.5% or more, or about 50% or more).

The HDL-C level following the administration (e,g. after 4 weeks of treatment) of 600 mg of the CETP inhibitor, particularly Compound I, is increased by about 15% or more relative to pretreatment HDL-C level (e.g., about 17.5% or more, about 20% or more, about 22.5% or more, about 25% or more, about 27.5% or more, about 30% or more, about 32.5% or more, about 35% or more, about 37.5% or more, about 40% or more, about 425% or more, about 45% or more, about 47.5% or more, about 50% or more, about 52.5% or more, or about 55% or more).

The HDL-C level following the administration (e.g. after 4 weeks of treatment) of 900 mg of the CETP inhibitor, particularly Compound I, is preferably increased by about 20% relative to pretreatment HDL-C level (e.g., about 22.5% or more, about 25% or more, about 27.5% or more, about 30% or more, about 32.5% or more, about 35% or more, about 37.5% or more, about 40% or more, about 42.5% or more, about 45% or more, about 47.5% or more, about 50% or more, about 52.5% or more, about 55% or more, about 57.5% or more, or about 60% or more).

Total cholesterol (TC) is determined using standard techniques known in the art. Preferably, the TC/HDL-C ratio following the administration (e.g. after 4 weeks of treatment) of 300 mg of the CETP inhibitors, particularly Compound I, is decreased by about 5% or more relative to the pretreatment TC/HDL-C ratio (e.g., about 7.5% or more, about 10% or more, about 12.5% or more, about 15% or more, about 17.5% or more, about 20% or more, about 22.5% or more, about 25% or more, about 27,5% or more, about 30% or more, about 32.5% or more, or about 35% or more).

The TC/HDL-C ratio following the administration (e.g. after 4 weeks of treatment) of 600 mg of the CETP inhibitor, particularly Compound I, is decreased by about 10% or more relative to the pretreatment TC/HDL-C ratio (e.g., about 12.5% or more, about 15% or more, about 17.5% or more, about 20% or more, about 22.5% or more, about 25% or more, about 27.5% or more, about 30% or more, about 32.5% or more, about 35% or more, or about 37.5% or more, or about 40% or more).

The TC/HDL-C ratio following the administration (e.g. after 4 weeks of treatment) of 900 mg of the CETP inhibitor, particularly Compound I, is decreased by about 15% or more relative to the pretreatment TC/HDL-C ratio (e.g., about 17.5% or more, about 20% or more, about 22.5% or more, about 25% or more, about 27.5% or more, about 30% or more, about 32.5% or more, about 35% or more, or about 37.5% or more, about 40% or more, about 42.4% or more, or about 45% or more).

Moreover, the invention provides a kit comprising a pharmaceutical composition comprising a therapeutically effective amount of a CETP inhibitor (e.g., Compound I) and a water-insoluble concentration-enhancing additive, prescribing information, and a container. The prescribing information can be prescribing information conforming to the methods of the invention and/or as otherwise discussed herein. The prescribing information preferably includes advice to a patient regarding the administration of the CETP inhibitor (e.g., Compound 1) with food, especially to improve the bioavailability of the CETP inhibitor.

In the following preferred embodiments are summarized:
1. A Pharmaceutical composition comprising a cholesteryl ester transfer protein inhibitor and crospovidone.
2. The pharmaceutical composition of item 1, wherein a major portion of the cholesterol ester transfer protein is crystalline.
3. The pharmaceutical composition of item 1, wherein the cholesteryl ester transfer protein is substantially crystalline.
4. The pharmaceutical composition of item 1, wherein the cholesteryl ester transfer protein is crystalline.
5. A Pharmaceutical composition comprising a substantially crystalline cholesteryl ester transfer protein inhibitor and a water-insoluble concentration-enhancing additive, wherein the cholesterol ester transfer protein inhibitor has the structure of Formula I 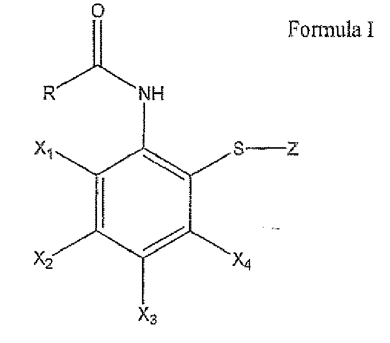 or a prodrug, compound, pharmaceutically acceptable salt, enantiomer, stereoisomer, hydrate, or solvate thereof, in which
   R represents
      a substituted or unsubstituted C₃₋₁₀ cycloalkyl group or a substituted or
      unsubstitued C₅₋₈ cycloalkenyl group;
   each of X₁, X₂, X₃, and X₄ may be the same or different and represents
      a hydrogen atom;
      a halogen atom;
      a C₁₋₄ alkyl group; a halo-C₁₋₄ alkyl group;
      a C₁₋₄ alkoxy group;
      a cyano group;
      a nitro group;
      an acyl group; or
      an aryl group; and
   Z represents
      a hydrogen atom;
      -YR₁, wherein
         Y represents -CO- or -CS-, and
         R₁ represents
            a substituted or unsubstitued straight chain or branched C₁₋₁₀ alkyl group;
            a C₁₋₄ alkoxy group;
            a C₁₋₄ alkylthio group;
            a substituted or unsubstituted amino group;
            a substituted or unsubstituted ureido group;
            a substituted or unsubstituted C₃₋₁₀ cycloalkyl group;
            a substituted or unsubstituted C₃₋₁₀ cycloalkyl C₁₋₁₀ alkyl group;
            a substituted or unsubstituted aryl group;
            a substituted or unsubstituted aralkyl group;
            a substituted or unsubstituted arylalkenyl group;
            a substituted or unsubstituted arylthio group;
            a substituted or unsubstituted 5- or 6-membered heterocyclic group having 1-3 nitrogen, oxygen, or sulfur atoms; or
            a substituted or unsubstituted 5- or 6-menabered heteroarylaklyl group; or
      -S-R₂, wherein
         R₂ represent
            a substituted or unsubstituted C₁₋₄ alkyl group or
               a substituted or unsubstituted aryl group.
6. The composition of item 5, wherein the cholesterol ester transfer protein inhibitor is crystalline.
7. The composition of item 5, wherein the cholesterol ester transfer protein inhibitor and water-insoluble concentration-enhancing additive are in a weight ratio of about 2:1 to about 9:1.
8. The composition of item 7, wherein the water-insoluble concentration-enhancing additive is crospovidone.
9. The composition of item 5, wherein the cholesteryl ester transfer protein inhibitor is a compound selected from the group consisting of
   N-(2-mercaptophenyl)-1-isopentylcyclohexanecarboxamide;
   N-(2-mercaptophenyl)-1-msthylcyclohexanecarboxamide;
   N-(2-mercaptophenyl)-1-isopentylcyclopentanecarboxamide;
   N-(2-mercaptophenyl)-1-isopropylcyclohexanecarboxamide;
   N-(4,5-dichloro-2-mercaptophenyl)-1-isopentylcyclohexanecarboxamide;
   N-(4,5-dichloro-2-mercaptophenyl)-1-isopentylcyclopentanecarboxamide;
   N-(2-mercapto-5-methylphenyl)-1-isopentylcyclohexanecarboxamide;
   N-(2-mercapto-4-methylphenyl)-1-isopentylcyclohexanecarboxamide;
   S-[2-(1-isopentylcyclohexanecarbonylamino)phenyl]thioacetate;
   S-[2-(1-methylcyclohexanecarbonylamino)phenyl] 2,2-dimethylthiopropionate;
   S-[2-(1-isopentylcyclohexanecarbonylamino)phenyl]2,2-dimethylthiopropionate;
   S-[2-(1-isopentylcyclohexanecarbonylamino)phenyl]2-acetylamino-3-phenylthiopropionate;
   S-[2-(1-isopentylcyclohexanecarbonylamino)phenyl]3-pyridinethiocarboxylate;
   S-[2-(1-isopentylcyclohexanecarbonylamino)phenyl]chlorothioacetate;
   S-[2-(1-isopentylcyclohexanecarbonylamino)phenyl]methoxythioacetate;
   S-[2-(1-isopentylcyclohexanecarbonylamino)phenyl]thiopropionate;
   S-[2-(1-isopentylcyclohexanecarbonylamino)phenyl]phenoxythioacetate;
   S-[2-(1-isopentylcyclohexanecarbonylamino)phenyl]2-methylthiopropionate;
   S-[2-(1-isopentylcyclohexanecarbonylainino)phenyl]4-chlorophenoxythioacetate;
   S-[2-(1-isopentylcyclohexanecarbonylamino)phenyl]cyclopropanethiocarboxylate;
   S-[2-(1-isopentylcyclohexanecarbonylamino)phenyl]2-acetylamino-4-carbamoylthiobutyrate;
   S[2-(1-isopentylcyclohexanecarbonylamino)phenyl]2-hydroxy-2-metylthiopropionate;
   S-[2-(1-isopentylcyclopentanecarbonylamino)phenyl]2,2-dimethylpropionate;
   2-[2-(1-isopentylcyclopentanecarbonylamino)phenyl]thioacetate;
   S-[4,5-dichloro-2-(1-isopentylcyclohexanecarbonylamino)phenyl] 2,2-dimemylthiopropionate;
   S-[4,05-dichloro-2-(1-isopentylcyclopentanecarbonylamino)phenyl]2,2-dimethythiopropionate;
   S-[2-(1-isopentylcylohexanecarbonylamino)-4-trifluoromethylphenyl] 2,2dimethylthiopropionate;
   O-methyl S-[2-(1-isopentylcyclohexanecarbonylamino)phenyl]monothiocarbonate;
   S-[2-(1-methylcyclohexanecarbonylamino)phenyl]S-phenyl dithiocarbonate;
   S-[2-(1-isopentylcyclohexanecarbonylamino)phenyl]N-phenylthiocarbamate;
   S-[4,5-dichloro-2-(1-cyclopropylcyclohexanecarbonylamino)phenyl]2,2-dimethylthiopropionate;
   S-[4,5-dichloro-2-(1-pentyltyclohexanecarbonylamino)phenyl]2,2-dimethylthiopropionate;
   S-[4,5-dichloro-2-(1-cyclopropylmethylcyclohexanecarbonylamino)phenyl]2,2-dimethylthiopropionate;
   S-[4,5-dichloro-2-(1-cyclohexylmethylcyclohexanecarbonylamino)phenyl]2,2-dimethylthiopropionate;
   S-[4,5-dichloro-2-(1-isopropylcyclohexanecarbonylamino)phenyl]2,2-dimethylthiopropionate;
   S-[4,5-dichloro-2-(1-isopentylcycloheptanecarbonylamino)phenyl]2,2-dimethylthiopropionate;
   S-[4,5-dichloro-2-(1-isopentylcyclobutanecarbonylamino)phenyl]2,2-dimethylthiopropionate;
   S-[2-(1-isopentylcyclohexanecarbonylanmino)-4-nitrophenyl]2,2-dimethylthiopropionate;
   S-[4-cyano-2-(1-isopentylcyclohexanecarbonylamino)phenyl]2,2-dimethylthiopropionate;
   S-[4-chloro-2-(1-isopentylcyclohexanecarbonylamino)phenyl]2,2-dimethylthiopropionate;
   S-[5-chloro-2-(1-isopentylcyclohexanecarbonylanino)phenyl]2,2-dimethylthiopropionate;
   S-[4-fluoro-2-(1-isopentylcyclohexanecarbonylamino)phenyl]2,2-dimethylthiopropionate;
   S-[4,5-difluoro-2-(1-isopentylcyclohexanecarbonylamino)phenyl]2,2-dimethylthiopropionate;
   S-[5-fluoro-2-(1-isopentylcyclohexanecarbonylamino)phenyl]2,2-dimethylthiopropionate;
   N-(2-mercaptophenyl)-1-ethylcyclohexanecarboxamide;
   N-(2-mercaptophenyl)-1-propylcyclohexanecarboxamide,
   N-(2-mercaptophenyl)-1-butylcyclohexanecarboxamide;
   N-(2-mercaptophenyl)-1-isobutylcyclohexanecarboxamide;
   S-[2-(1-isopentylcyclohexanecarbonylamino)phenyl]cyclohexanethiocarboxylate;
   S-[2-(1-isopentylcyclohexanecarbonylamino)phenyl]thiobenzoate;
   S-[2-(1-isopentylcyclohexanecarbonylamino)phenyl]5-carboxythiopentanoate;
   S-[2-(1-isopentylcyclohexanecarbonylamino)-4-methylphenyl] thioacetate;
   N-(2-mercaptophenyl)-1-(2-ethylbutyl)cyclohexanecarboxamide;
   S-[2-([[1-(2-ethylbutyl)cyclohexyl]carbonyl]amino)phenyl]2-methylpropanethioate;
   S-[2-(1-isobutylcyclohexanecarbonylamino)phenyl]2-methylthiopropionate;
   S-[2-[1-(2-ethylbutyl)cyclohexanecarbonylamino]phenyl]1-acetylpiperidine-4-thiocarboxylate;
   S-[2-[1-(2-ethylbutyl)cyclohexanecarbonylamino]phenyl]thioacetate;
   S-[2-[1-(2-ethylbutyl)cyclohexanecarbonylamino]phenyl]2,2-dimethylthiopropionate;
   S-[2-[1-(2-ethylbutyl)cyclohexanecarbonylamino]phenyl]methoxythioacetate;
   S-[2-[1-(2-ethylbutyl)cyclohexanecarbonylamino]phenyl]2-hydroxy-2-methylpropionate;
   S-[2-[1-(2-ethylbutyl)cyclohexanecarbonylamino]phenyl]4-chlorophenoxythioacetate;
   S-[2-(1-isobutylcyclohexanecarbonylamino)phenyl]4-chlorophenoxythioacetate; and
   S-[2-(1-isobutylcyclohexanecarbonylamino)phenyl]1-acetylpiperidine-4-thiocarboxylate; or a prodrug compound, a pharmaceutically acceptable salt, a hydrate, or a solvate thereof
10. The composition of item. 5, wherein the cholesteryl ester transfer protein inhibitor is a prodrug that forms *S*-[2-([[1-(2-ethylbutyl)cyclohexyl]carbonyl]amino)phenyl] thiol in viva.
11. The composition of item 5, wherein the cholesteryl ester transfer protein inhibitor is *S*-[2-([[1-(2-ethylbutyl)cylclohexyl]carbonyl]amino)phenyl] 2-methylpropanethioate.
12. The composition of item 11, wherein the cholesteryl ester transfer protein inhibitors is crystalline.
13. The composition of item 11, wherein the cholesterol ester transfer proteiza - inhibitor and the water-insoluble concentration-enhancing additive are in a weight ratio of about 2:1 to about 9:1.
14. The composition of item 13, wherein the water-insoluble concentration-enhancing additive is crospovidone.
15. A method for the treatment or prophylaxis of a cardiovascular disorder in a mammal, which comprises administering to the mammal a therapeutically effective amount of a pharmaceutical composition of any one of items 1-14.
16. The method of item 15, wherein the cardiovascular disorder is selected from the group consisting of atherosclerosis, peripheral vascular disease, dyslipidemia, hyperbetalipoproteinemia, hypoalphalipoproteinemia, hypercholesterolemia, hypertriglyceridemia, familial-hyperclaolesterolemia, angina, ischemia, cardiac ischemia, stroke, myocardial infarction, reperfusion injury angioplastic restenosis, hypertension, and vascular complications of diabetes, obesity or endotoxemia.
17. The method of item 15, wherein the cardiovascular disorder is selected from the group consisting of cardiovascular disease, coronary heart disease, coronary artery disease, hypoalphalipoproteinemia, hyperbetalipoproteinemia, hypercholesterolemia, hyperlipidemia, atherosclerosis, hypertension, hypertriglyceridemia, hyperlipidoproteinemia, peripheral vascular disease, angina, ischemia, and myocardial infarction.
18. The method of item 15, wherein a maximum concentration of the cholesteryl ester transfer protein inhibitor, or active form thereof, in the bloodstream of a mammal is at least about 0.35 5 µg/mL post-treatment relative to pretreatment when the cholesteryl ester transfer protein inhibitor is *S*-[2-([[1-(2-ethylbutyl)cyclohexyl]carbonyl]ainino)phenyl] 2-methylpropanethioate administered at a daily dose of 600 mg with food.
19. The method of item 15, wherein a maximum concentration of the cholesteryl ester transfer protein inhibitor, or active form thereof, in the bloodstream of a mammal is at least about 0.8 µg/mL post-treatment relative to pretreatment when the cholesteryl ester transfer protein inhibitor is *S*-[2-([[1-(2-ethylbutyl)cyclohexyl]carbonyl]amino)phenyl] 2-methylpropanethioate administered at a daily dose of 900 mg with food.
20. The method of item 15, wherein an area under the plasma concentration-time curve AUC_{0-∞} of the cholesteryl ester transfer protein inhibitor, or active form thereof, in the bloodstream of a mammal is at least about 3.5 µg·h/mL post-treatment relative to pretreatment when the cholesteryl ester transfer protein inhibitor is *S*-[2-([[1 -(2-ethylbutyl)cyclohexyl]carbonyl]amino)phenyl]2-methylpropanethioate administered at a daily dose of 600 mg with food.
21. The method of item 15, wherein an area under the plasma concentration-time curve AUG_{0-∞} of the cholesteryl ester transfer protein inhibitor, or active form thereof, in the bloodstream of a mammal is at least about 7.5 µg · h/mL post treatment relative to pretreatment when the cholesteryl ester transfer protein inhibitor is *S*-[2-([[1-(2-ethylbutyl)cyclohexyl]carbonyl]amino)phenyl]2-methylpropataethioate administered at a daily dose of 900 mg with food.
22. The method of item 15, wherein cholesteryl ester transfer protein activity in the bloodstream of a mammal is inhibited post-treatment by at least about 25% relative to CETP activity pretreatment when the cholesteryl ester transfer protein inhibitor is *S*-[2-([[1-(2-ethylbutyl)cyclohexyl]carbonyl]amino)phenyl] 2-methylpropanethioate administered at a daily dose of 600 mg with food.
23. The method of item 15, wherein cholesteryl ester transfer protein activity in the bloodstream of a mammal is inhibited post-treatment by at least about 35% relative to CETP activity pretreatment when the cholesterol ester transfer protein inhibitor is *S*-[2-([[1-(2-ethylbutyl)cyclohexyl]carbonyl]amino)phenyl] 2-methylpropanethioate administered at a daily dose of 900 mg with food.

The following examples further illustrate fhe invention but, of course, should not be construed as in any way limiting its scope.

### EXAMPLE I

This example illustrates the absorption af a CETP inhibitor (e.g., Compound I) when administered in a pharmaceutical composition according to the invention.

For this study, Caucasian male human subjects were administered 100 mg, 300 mg, 600 mg, 900 mg, 1200 mg, 1500 mg, or 1800 mg of Compound I, or placebo, after a standard breakfast. The tablets administered at each dose level are recited in Table 1.

**Tables 1 - Tablets Administered at Each Dose Level**

| **Dose Level (mg)** | **Number of Tablets Administered per Subject** | |
|---|---|---|
| | **Compound I** | **Placebo** |
| 100 | 1 x 100 mg | 1 x placebo |
| 300 | 1 x 300 mg | 1 x placebo |
| 600 | 2 x 300 mg | 2 x placebo |
| 900 | 3 x 300 mg | 3 x placebo |
| 1200 | 4 x 300 mg | 4 x placebo |
| 1500 | 5 x 300 mg | 5 x placebo |
| 1800 | 6 x 300 mg | 6 x placebo |

The uncoated white tablets were prepared using standard tableting procedures. The 100 mg tablets comprised 100 mg of Compound I, 6 mg of hydroxypropylmethyl cellulose 2910 as a binder, 175.1 mg of microcrystalline cellulose and 116.8 mg of lactose as diluents, 18 mg talc and 1.2 mg of magnesium stearate as lubricants, and 39.9 mg of crospovidone and 90 mg of low substituted hydroxypropyl cellulose as disintegrants.

The 300 mg tablets comprised 300 mg of Compound I, 18 mg of hydroxypropylmethyl cellulose 2910 as a binder, 18 mg of talc and 1.2 mg of magnesium stearate as lubricants, and 119.8 mg of crospovidone and 90 mg of low substituted hydroxypropyl cellulose as disintegrants.

The placebo tablets comprised 175.1 mg of microrystalline cellulose and 262.7 mg of lactose as diluents, 18 mg of tale and 1.2 mg of magnesium stearate as lubricants, and 90 mg of low substituted hydroxypropyl cellulose as a disintegrant. The placebo tablets were similar in appearance to the tablets comprising Compound I.

Treatments were administered orally with 150 mL water while standing. Subjects were not allowed to lie supine for 2 hours after dose administration, except for study procedures.

Doses were administered at similar times for each subject in each treatment period. Dosing commenced at approximately 08:30 hours. All subjects fasted from food and fluids (with the exception of water) from 22:00 hours on the day prior to dosing (Day - 1) until breakfast on Day 1, and during the evening prior to the post-study visit until laboratory safety evaluations had been performed on the following day. Water could be consumed at any time during the study, with the exception of the period up to 2 hours post-dose, when no fluids were permitted.

Subjects received a standard breakfast about 45 minutes prior to dosing. The meal was eaten at a steady rate over a 15 minute period so that the meal was completed 30 minutes before dosing. The standard breakfast consisted of the following:
200 mL orange juice
Two packets of cereal (approximately 60 g)
Two slices of wholemeal toast
10 g low fat spread (one packet)
20 g jam (one packet)
242 mL full fat milk (approximately 250 g)
Total energy content: 711 Kcal
Total fat content: 15.72 g (19.9% of total calories)
Total protein: 20.82 g (11.7% total calories)

Blood samples for pharmacokinetic analysis were taken immediately prior to dosing and at the following times after dosing: 1,2, 4, 6, 7,10,12, 24, and 36 hours post-dose.

The following pharmacokinetic parameters were calculated for the different dosage profiles and are defined as follows:
tₘₐₓ Time of maximum observed plasma concentration of the active form of Compound I;
Cₘₐₓ Maximum observed plasma concentration of the active form of Compound I;
t_{1/2} Half-life of plasma concentration of the active form of Compound I;
AUC_{0-tz} Area under the plasma concentration-time curve (AUC) from time zero up to the last quantifiable concentration (0-t_{z}), of the active form of Compound I; and
AUC_{0-∞} AUC from time zero to infinity (0-∞)

Pharmacokinetic parameters were log-transformed by analysis and assessed using SAS® Least Square Means derived from a three-way analysis of variance (ANOVA) fitting effects for subject, treatment, and period. Treatment comparisons were made by calculating the difference and 95% confidence intervals (CIs) of fhe difference of the log SAS® Least Square Means between parameters for the respective treatments. The differences and CIs of the differences were back-transformed for reporting purposes.

The plasma concentration of the active form of Compound I was determined by the following assay. Plasma samples were isolated from patients treated with Compound I. The plasma samples were treated with sodium hydroxide (Wako Pure Chemical Industries, Ltd.) to convert active forms of Compound I in the plasma to the thiol form (i.e., Compound II), The plasma sample next was treated with dithiothreitol (DTT) (Wako Pure Chemical Industries, Ltd.) to prevent the oxidation of thiol groups (i.e., to maintain thiol groups in a reduced state). N-ethylmaleiznide (NEM) (Wako Pure Chemical Industries, Ltd.) was added to stabilize the thiol form (i.e., Compound II) by, it is believed, blocking the free sulfhydryl group by the derivatization to an NEM-adduct. The sample then was analysed using High Performance Liquid Chromatography (HPLC). Finally, the results of the HPLC analysis of the plasma sample were compared to a known standard to determine the plasma concentration of the active form of Compound I. The standard of known concentration was prepared essentially as described above, with the exception that human plasma was isolated from humans who were not treated with Compound I. These "blank plasma" samples were combined with a known amount of Compound I.

The mean test results for plasma pharmacokinetic parameters, AUC _{0-∞} (µg·h/mL), AUC _{0-∞} (µg·h/mL), Cₘₐₓ (µg/mL), t_{1/2} (h), and tₘₐₓ (h), of the active form of Compound I are summarized in Table 2.

**Tables 2 - Plasma Pharmacokinetic Parameters of the Active Form of S-[2-([[1-(2- ethylbutyl)cyclohexyl]carbonyl]amino)phenyl]2-methylpropanethioate**

| **Parameters** | **Dose of Compound I** | | | | | | |
|---|---|---|---|---|---|---|---|
| | 100 mg | 300 mg | 600 mg | 900 mg | 1200 mg | 1500 mg | 1800 mg |
| AUC (0-t_{z})* | NA | 0.735 | 4.08 | 7.71 | 18.5 | 22.4 | 30.4 |
| (µg·h/mL) | (NA) | (45.6) | (22.3) | (14.7) | (19.0) | (16.1) | (25.4) |
| AUC (0-∞)* | NC | NA | 4.61 | 8.74 | 21.1 | 24.7 | 34.9 |
| (µg·h/mL) | (NC) | (NA) | (19.0) | (15.9) | (16.4) | (14.9) | (21.6) |
| Cₘₐₓ * | 0.024 | 0.161 | 0.485 | 0.869 | 2.06 | 2.68 | 3.45 |
| (µg/mL) | (53.0) | (51.1) | (41.7) | (17.2) | (31.5) | (28.9) | (35.8) |
| tₘₐₓ^{†} (h) | 3.00 | 2.00 | 3.00 | 4.00 | 5.00 | 4.00 | 5.00 |
| | (0.500- | (2.00- | (2.00- | (2.00- | (4.00- | (4.00- | (4.00- |
| | 6.00) | 4.00) | 6.00) | 6.00) | 6.00) | 6.00) | 6.00) |
| Cₘₐₓ* | 0.018 | 0.040 | 0.059 | 0.071 | 0.121 | 0.129 | 0.151 |
| (norm) | (51.8) | (62.2) | (40.4) | (17.1) | (32.1) | (25.4) | (37.3) |
| AUC (0-t_{z})* | NA | 0.186 | 0.500 | 0.631 | 1.09 | 1.08 | 1.34 |
| (norm) | (NA) | (57.0) | (22.4) | (21.4) | (24.2) | (14.1) | (24.1) |
| AUC (0-∞)* | NC | NA | 0.566 | 0.715 | 1.24 | 1.19 | 1.53 |
| (norm) | (NC) | (NA) | (19.3) | (22.7) | (21.9) | (15.1) | (18.4) |
| t_{1/2} ^{‡} (h) | NC (NC) | NA (NA) | 11.8 | 13.1 | 12.8 | 11.1 | 12.4 |
| | | | (7.09- | (10.8- | (10.6- | (8.84- | (9.32- |
| | | | 17.2) | 14.9) | 16.4) | 14.5) | 20.3) |

| | | | | | | | |
|---|---|---|---|---|---|---|---|
| * = geometric mean (geometric coefficient of variation %) ^{†} = median (min-max) ^{‡} = harmonic mean (min-max) NA = not applicable NC = not calculable norm = normalized for dose and body weight (mg/kg) | | | | | | | |

As demonstrated by the data in Table 2, a pharmaceutical composition comprising a CETP inhibitor can achieve a maximum observed plasma concentration (Cₘₐₓ) of the CETP inhibitor, or active form thereof, in the bloodstream of a mammal of at least about 0.1 µg/mL at a dose of 300 mg when administered with food. For example, at a dose of 300 mg of Compound I, the Cₘₐₓ was about 0.16 ug/mL.

The data in Table 2 also demonstrates that a pharmaceutical composition comprising a CETP inhibitor can achieve a maximum observed plasma concentration (Cₘₐₓ) of the CETP inhibitor, or active form thereof, in the bloodstream of a mammal of at least about 0.35 µg/mL and an area under the plasma concentration-time curve (AUC_{0-∞}) of at least about 3.5 µg·h/mL, at a dose of 600 mg when administered with food. For example, at a dose of 600 mg of Compound I, the Cₘₐₓ was about 0.5 µg/mL, and the AUC_{0-∞} was about 5 µg·h/mL.

Additionally, the data indicates that a pharmaceutical composition comprising a CETP inhibitor can achieve a maximum observed plasma concentration (Cₘₐₓ) of at least about 0.8 µg/mL and an area under the plasma concentration-time curve (AUC_{0-∞}) of at least about 7.5 µg·h/mL, at a dose of 900 mg when administered with food. For example, at a dose of 900 mg of Compound I, the Cₘₐₓ was about 0.9 µg/mL, and the AUC_{0-∞} was about 9 µg·h/mL.

### EXAMPLE 2

The effect of food on the absorption of a CETP inhibitor in patients was identified in a study designed to compare the bioavailability of 900 mg of Compound I orally administered to Caucasian male volunteers with and without food.

For this study each of six subjects received Compound I at a dose level of 900 mg in each of two treatment periods, once with food (after a standard breakfast) and once in the fasted state. There was a minimum of 7 days between each treatment period. This interval of 7 days between treatments was considered appropriate for eliminating any within-subject carryover effects.

The subjects received 900 mg of Compound I by administration of 3 tablets of 300 mg each. Tablet preparation and administration procedures were as described as in Example 1, with the following exceptions.

Doses were administered at similar times for each subject in each treatment period. Dosing commenced at approximately 08:30 hours. All subjects fasted from food and fluids (with the exception of water) from 22:00 hours on the day prior to dosing (Day 1) until breakfast on Day 1 (for subjects receiving Compound I in the fed state (i.e., with food)) or lunch-time on Day 1 (for subjects receiving Compound I in the fasted state), and during the evening prior to the post-study visit until laboratory safety evaluations had been performed on the following day. Water could be consumed at any time during the study, with the exception of the period up to 2 hours post-dose, when no fluids were permitted.

When subjects were administered Compound I in the fed state, they received a standard breakfast about 45 minutes prior to dosing as described in Example 1.

The mean test results for plasma pharmacokinetic parameters; AUC _{0-∞} (µg·h/mL), AUC _{0-tz} (µg·h/mL), Cₘₐₓ (µg/mL), t_{1/2}(h), and tₘₐₓ (h), of the active form of Compound I are summarized in Table 3.

**Table 3 - Plasma Pharmacokinetic Parameters of the Active Form of S-[2-([[1-(2- ethylbutyl)cyclohexyl]carbonyl]amino)phenyl]2-methylpropanethioate**

| **Parameter** | **Treatment Protocol** | | **Ratio** |
|---|---|---|---|
| | **Fasted** | **Fed** | **(Fed:Fasted)** |
| AUC (0-t_{z})* | 6.21 | 10.2 | 1.65 |
| (µg·h/mL) | (46.9) | (19.0) | |
| AUC (0-∞) * | 7.97 | 12.5 | 1.57 |
| (µg·h/mL) | (46.7) | (17.4) | |
| Cₘₐₓ * | 0.423 | 0.955 | 2.26 |
| (gl/mL) | (37.1) | (26.1) | |
| tₘₐₓ (h)^{†} | 5.00 | 4.00 | NA |
| | (2.00-6.00) | (2.00-6.00) | |
| t_{1/2} (h)^{‡} | 16.5 | 15.4 | 0.935 |
| | (14.1-22.4) | (12.6-18.6) | |

| | | | |
|---|---|---|---|
| * = geometric mean (geometric coefficient of variation %) ^{†} = median (min-max) NA = not applicable ^{‡} = harmonic mean (min-max) | | | |

The absorption of the active form of Compound I was relatively slow, with the time of maximum observed plasma concentration occurring at between 4 and 5 hours after administration of Compound I. As is apparent from Table 3, the time of maximum observed plasma concentration was similar after administration of Compound I with and without food. Additionally, the half life of the active form of Compound I was determined to be similar after administration of the drug with and without food.

Several of the phamacokinetic parameters, however, were affected by the administration of Compound I with food. These include AUC_{0-tz}, AUC _{0-∞}, and Cₘₐₓ, which were 65%, 57%, and 126% higher, respectively, when Compound I was administered with food as compared with the administration of Compound I in the fasted state. These increases are noticeably apparent when the geometric mean plasma concentrations of the active form of Compound I were plotted in linear form in Figure 1 and plotted in semi-logarithmic form in Figure 2.

The observed increases in pharmacokinetic parameters when Compound is administered With food indicate an increase in the bioavailability of the active form of the drug when compared to administration of the drug under fasted conditions.

Therefore, this example demonstrates that an increase in bioavailability of a CETP inhibitor results when the CETP inhibitor is administered with food relative to administration without food.

### EXAMPLE 3

This example further illustrates the absorption of a CETP inhibitor (e.g., Compound I) when administered in a pharmaceutical composition according to the invention.

Japanese male human subjects were administered 100 mg, 300 mg, 600 mg, 900 mg, 1200 mg, 1500 mg, or 1800 mg of Compound I, or placebo, following a standard breakfast. Administration, dosing, and sampling schedules were commensurate with those described in Example 1. The tablets were prepared as described in Example 1.

The mean test results for plasma pharmacokinetics parameters of the active form of Compound I, AUC_{0-∞} (µg·h/mL), Cₘₐₓ (µg/mL), and tₘₐₓ (h), as well as t_{1/2} α (h) and t_{1/2} β, are summarized in Table 4. t_{1/2} α signifies the half-life in the α-phase of plasma concentration of the active form of Compound I, and t_{1/2} β signifies the half-life in the β-phase of plasma concentration of the active form of Compound I.

**Table 4 - Plasma Pharmacoknetic Parameters of the Active Form or S-[2-([[1-(2- ethylbutyl)cyclohexyl]carbonyl]amino)phenyl]2- methylpropanethioate**

| **Parameter** | **Dose of Compound I** | | | | | | |
|---|---|---|---|---|---|---|---|
| | 100 mg | 300 mg | 600 mg | 900 mg | 1200 mg | 1500mg | 1800 mg |
| AUC (0-∞) (µg·h/mL) | 0.120 ± | 2.133 ± | 10.458 ± | 14.936 ± | 24.197 ± | 43.062 ± | 40.057 ± |
| | 0.025 | 0.846 | 2.837 | 5.113 | 8.964 | 14.923 | 12.319 |
| | (1.0) | (17.8) | (87.2) | (124.5) | (201.6) | (358.9) | (333.8) |
| Cₘₐₓ (µg/mL) | 0.038 ± | 0.254 ± | 1.029 ± | 1.716 ± | 2.957 ± | 5.467 ± | 5.115 ± |
| | 0.013 | 0.080 | 0.378 | 0.521 | 1.136 | 2.227 | 1.550 |
| | (1.0) | (6.7) | (27.1) | (45.2) | (77.8) | (143.9) | (134.6) |
| tₘₐₓ (h) | 2.3 ± 0.8 | 2.3 ± 0.8 | 4.3 ± 1.5 | 2.7 ± 1.0 | 4.0 ± 0.0 | 4.3 ± 0.8 | 2.7 ± 1.0 |
| t_{1/2}α (h) | - | 2.3 ± 0.4 | 2.7 ± 0.6 | 3.1 ± 0.3 | 2.7 ± 0.4 | 2.8 ± 0.4 | 2.6 ± 0.5 |
| t_{1/2} β (h) | - | 13.7 ± | 15.1 ± | 15.7 ± | 11.8 ± | 12.2 ± | 11.9 ± |
| | | 5.1 | 2.1 | 2.1 | 3.7 | 1.5 | 1.9 |

As demonstrated by the data in Table 4, a pharmaceutical composition comprising a CETP inhibitor can achieve a maximum observed plasma concentration (Cₘₐₓ) of the CETP inhibitor, or active form thereof, in the bloodstream of a mammal of at least about 0.1 µg/mL and an area under the plasma concentration-time curve (AUC_{0-∞}) of at least about 0.5 µg·h/mL, at a dose of 300 mg when administered with food. For example, at a dose of 300 mg of Compound I, the Cₘₐₓ was about 0.2 µg/mL, and the AUC_{0-∞} was about 2 µg·h/mL.

The data in Table 4 also demonstrates that a pharmaceutical composition comprising a CETP inhibitor can achieve a maximum observed plasma concentration (Cₘₐₓ) of at least about 0.35 µg/mL and an area under the plasma concentration-time curve (AUC_{0-∞}) of the CETP inhibitor, or active form thereof, in the bloodstream of a mammal of at least about 3.5 µg·h/mL, at a dose of 600 mg when administered with food. For example, at a dose of 600 mg of Compound I, the Cₘₐₓ was about 1 µg/mL, and the AUC_{0-∞} was about 10 µg·h/mL.

Additionally, the data indicates that a pharmaceutical composition comprising a CETP inhibitor can achieve a maximum observed plasma concentration (Cₘₐₓ) of at least about 0.8 µg/mL and an area under the plasma concentration-time curve (AUC_{0-∞}) of at least about 7.5 µg·h/mL, at a dose of 900 mg when administered with food. For example, at a dose of 900 mg of Compound I, the Cₘₐₓ was about 1.7 µg/mL, and the AUC_{0-∞} was about 15 µg·h/mL.

### EXAMPLE 4

In a similar study to that described in Example 2, the effect of food on the absorption of the active form of Compound I in patients was identified in a study designed to compare the bioavailability of 600 mg of Compound I orally administered to Japanese male volunteers with and without food.

Administration, dosing, and sampling schedules were commensurate with those described in Example 2. However, patients were administered 600 mg (rather than 900 mg) of Compound I with and without food. Patients were administered two tablets of 300 mg each. The tablets were prepared as described in Example 1.

The mean test results for plasma pharmacokinetics parameters of the active form of Compound I, AUC _{0-∞} (µg·h/mL), Cₘₐₓ (µg/mL), and tₘₐₓ (h), as well as t_{1/2} α (h) and t_{1/2} β, are summarized in Table 5.

**Table 5 - Plasma Pharmacokinetic Parameters of the Active Form of S-[2-([[1-(2- ethylbutyl)cyclohexyl]carbonyl]amino)phenyl] 2-methylpropanethioate**

| **Parameter** | **Treatment Protocol** | |
|---|---|---|
| | **Fasted** | **Fed** |
| AUC (0-∞) | 5.395 ± 1.413 | 10.458 ± 2.837 |
| (µg h/mL) | (0.52) | (1.00) |
| Cₘₐₓ | 0.316 ± 0.061 | 1.029 ± 0.378 |
| (µg/mL) | (0.31) | (1.00) |
| tₘₐₓ (h) | 2.2 ± 1.1 | 4.3 ± 1.5 |
| t_{1/2}α (h) | 5.5 ± 1.4 | 2.7 ± 0.6 |
| t_{1/2β} (h) | 20.6 ± 3.0 | 15.1 ± 2.1 |

Pharmacokinetics parameters, such as maximum observed plasma concentration (Cₘₐₓ) and area under the plasma concentration-time curve from time zero to infinity (AUC_{0-∞}), were affected by administration of Compound I with food. The Cₘₐₓ value after administration of 600 mg of Compound I was 1.029 µg/mL when administered with food and only 0.316 µp g/mL when administered without food. The AUC_{0-∞} value after administration of 600 mg of Compound I was 10.458 µg h/mL when administered with food and only 5.395 µg 1h/mL when administered without food. Thus, the Cₘₐₓ and AUC_{0-∞} were about 3 and 2 times higher, respectively, when the patients were administered the CETP inhibitor with food as compared to without food.

The observed increases in the pharmacokinetic parameters when Compound I is administered with food indicate that the active form of the drug is more readily absorbed when administered with food, such as after a meal. Thus, the administration of a CETP inhibitor (e.g., Compound I) with food results in an increase in the bioavailability of the active form of the drug relative to the administration of the CETP inhibitor under fasted conditions.

### EXAMPLE 5

This example illustrates the effect of the administration of a CETP inhibitor (e.g., Compound I) on CETP activity when administered in a pharmaceutical composition according to the invention.

Caucasian male human patients were orally administered 100 mg, 200 mg, 600 mg, 900 mg, 1200 mg, 1500 mg, 1800 mg of Compound I, or placebo, following breakfast. Administration, dosing, and sampling schedules were substantially similar to those described in Example 1. The tablets were prepared as described in Example 1.

The procedure for determining CETP activity was substantially similar to the procedures described in Tollefson et al., Methods Enzymol., 129, 797-816 (1986), and Kato et al., J. Biol. Chem., 264, 4082-4087 (1989).

CETP activity and changes from baseline (pre-dose) were measured, and the resulting data is summarized in Table 6 as percentage change from baseline.

**Table 6 - Mean (S.D.) Changes from Baseline (Pre-dose) in CETP Activity**

| **Treatment** | **Predose** | **Percent Changes from Pre-dose (standard deviation)** | | | | | | |
|---|---|---|---|---|---|---|---|---|
| | | **1 h** | **2 h** | **4 h** | **6 h** | **8 h** | **24 h** | **Post-study** |
| Placebo | 90 | -2 | -1 | 0 | -3 | -3 | 2 | 0 |
| | (15.8) | (2.4) | (3.1) | (3.1) | (3.0) | (4.0) | (4.4) | (8.9) |
| 100 mag | 104 | -2 | -2 | -5 | -7 | -6 | -1 | -5 |
| Compound I | (15.4) | (3.8) | (3.4) | (5.6) | (3.0) | (4.0) | (5.3) | (2.2) |
| 300 mg | 88 | 0 | -7 | -12 | -13 | -13 | -6 | 2 |
| Compound I | (10.4) | (2.1) | (3.3) | (4.3) | (3.4) | (3.4) | (3.5) | (6.1) |
| 600 mg | 92 | -3 | -12 | -29 | -36 | -36 | -21 | -6 |
| Compound I | (22.9) | (4.0) | (5.9) | (13.1) | (13.4) | (14.6) | (10.3) | (8.9) |
| 900 mg | 90 | -3 | -23 | -48 | -55 | -53 | -28 | -5 |
| Compound I | (17.2) | (3.8) | (14.1) | (11.8) | (9.9) | (9.2) | (5.2) | (10.8) |
| 1200 mg | 88 | -3 | -17 | -58 | -71 | -70 | -43 | -4 |
| Compound I | (5.8) | (2.4) | (9.8) | (6.3) | (4.7) | (5.4) | (5.7) | (8.8) |
| 1500 mg | 96 | -3 | -32 | -72 | -83 | -81 | -48 | 0 |
| Compound I | (12.4) | (2.6) | (21.6) | (21.1) | (9.7) | (9.0) | (6.1) | (49) |
| 1800 mg | 82 | -3 | -23 | -67 | -74 | -71 | -42 | -5 |
| Compound I | (16.0) | (3.7) | (14.7) | (16.2) | (15,2) | (14.3) | (10.9) | (5.1) |

As demonstrated by the data in Table 6, a pharmaceutical composition comprising a CETP inhibitor can achieve a decrease in CETP activity of at least about 10% relative to pre-dose levels at a dose of 300 mg. For example, 6 hours after the administration of 300 mg of Compound I, CETP activity had decreased by 13%.

The data in Table 6 also demonstrates that a pharmaceutical composition comprising a CETP inhibitor can achieve a decrease in CETP activity of at least about 25% relative to pre-dose levels at a dose of 600mg. For example, 6 hours after the administration of 600 mg of Compound I, CETP activity had decreased by 36%.

Additionally, the data indicates that a pharmaceutical composition comprising a CETP inhibitor can achieve a decrease in CETP activity of at least about 35% relative to placebo at a dose of 900 mg. For example, 6 hours after the administration of 900 mg of Compound I, CETP activity had decreased by 55%.

### EXAMPLE 6

The effect of food on the absorption of the active form of Compound I in Caucasian male patients was identified in a study designed to compare the CETP activity following the oral administration of 900 mg of Compound I with and without food.

Administration, dosing, and sampling schedules were substantially similar to those described in Example 1.

The procedure for determining CETP activity is described in Example 5.

CETP activity and changes from baseline (pre-dose) over time were measured, and the resulting data is summarized in Table 7. The mean changes from baseline (pre-dose) in CETP activity over time are set out in the plot of Figure 3.

**Table 7 - Mean (S.D.) Changes from Baseline (Pre-dose) in CETP Activity**

| **Treatment Protocol** | **Pre- dose** | **Percent Changes from Pre-dose (standard deviation)** | | | | | | |
|---|---|---|---|---|---|---|---|---|
| | | **1 h** | **2 h** | **4 h** | **6 h** | **8 h** | **24 h** | **Post- study (all subjects)** |
| **Fed** | 96 | -1 | -16 | -44 | -59 | -58 | -34 | |
| | (17.0) | (2.3) | (13.2) | (14,0) | (11.1) | (11.1) | (7.5) | 96 |
| **Fasted** | 91 | 1 | 2 | -4 | -10 | -15 | -10 | (17.4) |
| | (16.0) | (2.2) | (2.4) | (2.2) | (3.1) | (4.7) | (3.8) | |

A clear difference in CETP activity was observed when Compound I was administered with and without food. Inhibition of CETP activity was much more marked in the fed treatment protocol as compared with the fasted treatment protocol. For example, between 4 and 24 hours post-dose, there was a significant decrease in CETP activity in the fed versus the fasted state. Such a decrease in CETP activity indicates increased bioavailability of the active form of the drug when administered with food as compared to administration of the drug without food.

The relationships between plasma concentrations of the active form of Compound I and inhibition of CETP activity for the fed and fasted states are illustrated by the plots of Figures 4 and 5, respectively. As plasma concentrations of the active form of Compound I increased, the inhibitory effect on CETP increased (i.e., CETP activity decreased).

### EXAMPLE 7

This example further illustrates the effect of the administration of a CETP inhibitor (e.g., Compound I) on the CETP activity when administered in a Pharmaceutical composition according to the invention.

Japanese male human patients were orally administered 100 mg, 300 mg, 600 mg, 900 mg, 1200 mg, 1500 mg, or 1800 mg of Compound I, or placebo, following breakfast.

Administration, dosing, and sampling schedules were commensurate with those described in Example 1. The tablets were prepared as described in Example 1.

Relative CETP activities (calculated as a percentage of baseline CETP activity) and standard deviations (SD) were measured, and the resulting data is summarized in Table 8.

**Table 8 - Relative CETP Activity**

| **Treatment** | **Pre- dose** | **Percent CETP Activity Relative to Pre-dose (standard deviation)** | | | | | | |
|---|---|---|---|---|---|---|---|---|
| | | **1 h** | **2 h** | **4 h** | **6 h** | **8 h** | **24 b** | **Post-study** |
| Placebo | 100 | 97.0 | 95.7 | 96.4 | 93.5 | 93.3 | 97.2 | 101.6 |
| | (0.0) | (3.4) | (4.3) | (3.8) | (3.1) | (4.0) | (5.7) | (9.6) |
| 100 mg | 100 | 96.2 | 92.0 | 93.2 | 90.4 | 90.4 | 99.2 | 99.2 |
| | (0.0) | (1.9) | (3.3) | (2.5) | (2.8) | (2.2) | (4.7) | (11.1) |
| 300 mg | 100 | 100.2 | 90.7 | 83.1 | 80.5 | 80.6 | 88.8 | 96.1 |
| | (0.0) | (3.9) | (5.5) | (4.2) | (3.9) | (5.6) | (3.8) | (9.9) |
| 600 mg | 100 | 100.4 | 87.8 | 52.6 | 37.6 | 39.1 | 65.5 | 102.9 |
| | (0.0) | (1.9) | (9.2) | (13.4) | (6.6) | (8.3) | (5.7) | (7.3) |
| 900 mg | 100 | 100.1 | 52.1 | 24.0 | 24.1 | 29.8 | 60.5 | 95.1 |
| | (0.0) | (2.9) | (9.3) | (9.0) | (8.1) | (7.9) | (7.6) | (5.6) |
| 1200 mg | 100 | 94.2 | 54.0 | 12.1 | 10.5 | 14.7 | 47.6 | 95.2 |
| | (0.0) | (2.0) | (17.0) | (5.5) | (3.6) | (4.3) | (8.2) | (8.8) |
| 1500 mg | 100 | 100.8 | 85.7 | 13.3 | 10.6 | 15.4 | 53.7 | 96.9 |
| | (0.0) | (3.3) | (13.2) | (7.9) | (1.7) | (2.1) | (5.7) | (6.8) |
| 1800 mg | 100 | 85.3 | 15.6 | 6.0 | 8.4 | 12.2 | 49.5 | 93.0 |
| | (0.0) | (11.5) | (8.7) | (1.7) | (0.9) | (1.4) | (3.0) | (6.6) |

As demonstrated by the data in Table 8, a pharmaceutical composition comprising a CETP inhibitor can achieve a decrease in CETP activity of at least about 10% relative to pre-dose levels at a dose of 300 mg. For example, 6 hours after the administration of 300 mg of Compound I, CETP activity is about 80.5% of the pre-dose value. Therefore, CETP activity has decreased by about 19.5% following the administration of Compound I.

The data in Table 8 also demonstrates that a pharmaceutical composition comprising a CETP inhibitor can achieve a decrease in CETP activity of at least about 25% relative to pre-dose levels at a dose of 600 mg. For example, 6 hours after the administration of 600 mg of Compound I, CETP activity is only about 38% of the pre-dose value. Therefore, CETP activity has decreased by about 62% following the administration of Compound I.

Additionally, the data indicates that a pharmaceutical composition comprising a CETP inhibitor can achieve a decrease in CETP activity of at least about 35% relative to pre-dose CETP activity at a dose of 900 mg. For example, 4 hours after the administration of 900 mg of Compound I, CETP activity is only about 24% of the pre-dose value. Thus, CETP activity has decreased by about 76%.

### EXAMPLE 8

In a similar study to that described in Example 6, the effect of food on the absorption of the active form of Compound I in Japanese male patients was identified in a study designed to compare the relative CETP activity following the oral administration of 600 mg of Compound I with and without food.

Administration, dosing, and sampling schedules were commensurate with those described in Examples 1 and 6. However, patients were administered 600 mg (rather than 900 mg as in Example 6) of Compound I with and without food. Patients were administered two tablets of 300 mg each. The tablets were prepared as described in Example 1.

Relative CETP activities (calculated as a percentage of baseline CETP activity) and standard deviations (SD) were measured, and the resulting data is summarized in Table 9.

**Table 9 - Relative CETP Activity**

| **Treatment Protocol** | **Pre-dose** | **CETP Activity Relative to Pre-dose (standard deviation)** | | | | | | |
|---|---|---|---|---|---|---|---|---|
| | | **1 h** | **2 h** | **4 h** | **6 h** | **8 h** | **24 h** | **Post-study** |
| **Fed** | 100 | 100.4 | 87.8 | 52.6 | 37.6 | 39.1 | 65.5 | 102.9 |
| | (0.0) | (1.9) | (9.2) | (13.4) | (6.6) | (8.3) | (5.7) | (7.3) |
| **Fasted** | 100 | 102.1 | 99.6 | 96.5 | 89.5 | 87.8 | 92.6 | 100.4 |
| | (0.0) | (3.6) | (2.1) | (3.1) | (2.0) | (4.6) | (3.0) | (2.8) |

A clear difference in CETP relative activity was observed when Compound I was administered with and without food, consistent with the results discussed in Example 2. Inhibition of CETP activity was much more marked in the fed treatment protocol as compared with the fasted treatment protocol. For example, between 4 and 24 hours post-dose, there was a significant decrease in CETP activity in the fed versus the fasted state. Specifically, the inhibition of CETP activity following the administration of Compound I with food reached its peak at 6 hours post-administration with 37.6% CETP activity relative to baseline. In contrast, the inhibition of CETP activity following the administration of Compound I without food reached its peak at 8 hours post-administration with 87.8% CETP activity relative to baseline. Such a decrease in the relative CETP activity following the administration of Compound I with food indicates increased bioavailability of the active form of the drug when administered with food as compared to the administration of the drug without food.

### EXAMPLE 9

This example illustrates the effect of the administration of Compound I on CETP activity and lipid levels in healthy individuals.

About 200 volunteers (men and women) were randomized to receive placebo or to receive 300 mg (low dose), 600 mg (medium dose) or 900 mg (high dose) of Compound I per day for 4 weeks. Each patient took three tablets after breakfast each day for 4 weeks. Patients either took three placebo tablets (placebo); one 300 mg tablet and two placebo tablets (low dose); two 300 mg tablets and one placebo tablet (medium dose); or three 300 mg tablets (high dose). Tablet preparation was as described as in Example 1.

The testing period consisted of (a) a run-in period of 4 weeks, followed by (b) 4 weeks of treatment, and (c) 4 weeks of monitoring. Blood samples were drawn after an overnight fast. For CETP activity assays, blood was drawn before Compound I intake and during and after treatment. HDL-C was determined with a heparin MnCl₂ precipitation reagent and LDL-C was calculated by the Friedewald formula *(see,* de Grooth et al., *supra).* CETP activity was measured as described in Example 5.

Table 10 describes the values of the assayed properties ((mean) ± standard deviation) at baseline (i.e., before administration of Compound I). Table 11 describes the absolute changes in the assayed properties after 4 weeks of treatment. The data points for absolute changes from baseline (i.e., before administration of Compound I) in CETP activity, total cholesterol (TC), HDL-C, LDL-C, and total cholesterol/HDL-C (TC/HDL-C) ratio are provided below. Analysis was done by fitting an ANOVA model with separate treatment effects for the four groups (i.e., placebo, 300 mg, 600 mg, or 900 mg of Compound I).

**Table 10 - Baseline Characteristics**

| **Assayed** | **Treatment Protocol** | | | |
|---|---|---|---|---|
| **Property** | **Placebo** | **300 mg** | **600 mg** | **900 mg** |
| | **(n=50)** | **(n=48)** | **(n=48)** | **(n=52)** |
| **CETP Activity (1% of control)** | 92.0±23.9 | 90.0±18.6 | 89.9±17.7 | 95.2±19.4 |
| **TC(mmol/L)** | 5.6±1.1 | 5.9±1.0 | 5.7±1.0 | 5.9±0.9 |
| **HDL-C (mmol/L)** | 1.16±0.23 | 1.16±0.20 | 1.21±0.25 | 1.16±0.24 |
| **LDL-C (mmol//L)** | 3.81±1.0 | 4.1±0.9 | 3.7±0.9 | 3.9±0.9 |
| **TC/HDL-C ratio** | 5.0±1.4 | 5.3±1.4 | 4.9±1.3 | 5.3±1.4 |

**Table 11 - Absolute Changes in Assayed Properties According to Dose of S-[2-([[1-(2-ethylbutyl)cyclohexyl]carbonyl]amino)phenyl]2-methylpropanethioate after 4 Weeks Treatment**

| **Assayed** | **Treatment Protocol** | | | |
|---|---|---|---|---|
| **Property** | **Placebo** | **300 mg** | **600 mg** | **900 mg** |
| | **(n=50)** | **(n=48)** | **(n=47)** | **(n=52)** |
| **CETP Activity (% of control)** | 0.9±13.2 | -15.4±11.9^{‡} | -29.6±19.5^{‡} | -37.2±17.6^{‡} |
| **TC (mmol/L)** | 0.0±0.5 | -0.1±0.5 | 0.0±0.6 | 0.0±0.6 |
| **HDL-C (mmol/L)** | 0.04±0.15 | 0.18±0.15^{†} | 0.32±0.22^{‡} | 0.40±0.29^{‡} |
| **LDL-C (mmol/L)** | -0.1±0.5 | -0.2±0.5 | -0.2±0.6 | -0.3±0.6* |
| **TC/HDL-C ratio** | -0.2±0.6 | -0.7±0.8^{†} | -0.9±0.8^{‡} | -1.2±0.7^{‡} |

| | | | | |
|---|---|---|---|---|
| * P≤0.01; ^{†} P≤0.001; ^{‡} P≤0.0001 (each group versus placebo) | | | | |

As demonstrated by the data in Tables 10 and 11, a pharmaceutical composition comprising a CETP inhibitor can achieve an increase in HDL-C levels of about 10%, about 15%, and about 20% at dose levels of 300 mg, 600 mg, and 900 mg of Compound I, respectively, following daily treatment for 4 weeks. For example, HDL-C levels were increased by about 15%, about 26%, and about 34% relative to baseline levels of the 300 mg, 600 mg, and 900 mg treatment groups, respectively.

Tables 10 and 11 also illustrate that the TC/HDL-C ratio can be decreased by about 5%, about 10%, and about 15% at dose levels of 300 mg, 600 mg, and 900 mg of Compound I, respectively, following daily treatment for 4 weeks. For example, the TC/HDL-C ratios were decreased by about 13%, about 18%, and about 23% relative to baseline levels of the 300 mg, 600 mg, and 900 mg treatment groups, respectively.

The data in Tables 10 and 11 also demonstrate that a pharmaceutical composition comprising a CETP inhibitor can achieve a decrease in CETP activity of at least about 10%, about 25%, and about 35% relative to pre-dose levels at a dose levels of 300 mg, 600 mg, or 900 mg of the CETP inhibitor (e.g., Compound I), respectively, following daily administration of the CETP inhibitor with food for 4 weeks. For example, CETP activity decreased by about 17%, about 33%, and about 39% relative to baseline levels of the 300 mg, 600 mg, and 900 mg treatment groups, respectively.

### EXAMPLE 10

The following example illustrates the method of manufacturing the formulation comprising 300 mg of Compound I described in Example 1.

In Step 1, Compound I was pulverized by jet mill. A particle size distribution of less than about 10 µm (e.g., about 5 µm) of pulverized Compound I was tested by the use of an in-process control.

In Step 2, the pulverized Compound I was mixed with crospovidone by drum mixer, resulting in a mixed powder.

In Step 3, the mixed powder of Step 2 was passed through a screen #12 approximately three times.

In Step 4, the screened, mixed powder of Step 3 was pre-mixed by wet granulator.

In Step 5, hydroxypropylmethylcellulose 2910 was dissolved in purified water using a propeller mixer.

In Step 6, the mixed powder of Step 4 was granulated using the solution of Step 5 as a binder in the wet granulator. This step yielded four batches of granulated material.

In Step 7, two of the four batches of granulated material of Step 6 were transferred to a fluidized bed dryer and dried. The process was repeated for the remaining two batches.

In Step 8, the granulated material of Step 7 was passed through a screen #22, Moisture content and particle size distribution were tested by an in-process control.

In Step 9, all of the dried granulated material of Step 8 was mixed by drum mixer.

In Step 10, the granulated material of Step 9 was mixed with low substituted hydroxypropyl cellulose by drum mixer to yield mixed granulated material.

In Step 11, the mixed granulated material of Step 10 was mixed with talc and magnesium stearate by drum mixer. Content uniformity, specific volume, and angle of repose were tested by an-process control.

In Step 12, the mixed granulated material was compressed by a tableting machine. Content uniformity, tablet hardness, thickness, and friability were assayed. Additionally, dissolution and weight variation tests were performed.

This example demonstrates that a formulation comprising 300 mg Compound I, hydroxypropylmethyl cellulose 29 10, talc, magnesium stearate, crospovidone, and low substituted hydroxypropyl cellulose (as described in Example 1) can be formed into an oral dosage form.

All references, including publications, patent applications, and patents, cited herein are hereby incorporated by reference to the same extent as if each reference were individually and specifically indicated to be incorporated by reference and were set forth in its entirety herein.

The use af the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A pharmaceutical composition comprising a cholesteryl ester transfer protein inhibitor and crospovidone.

2. A pharmaceutical composition comprising a cholesteryl ester transfer protein inhibitor and a water-insoluble concentration-enhancing additive, wherein the cholesteryl ester transfer protein inhibitor has the structure of Formula I 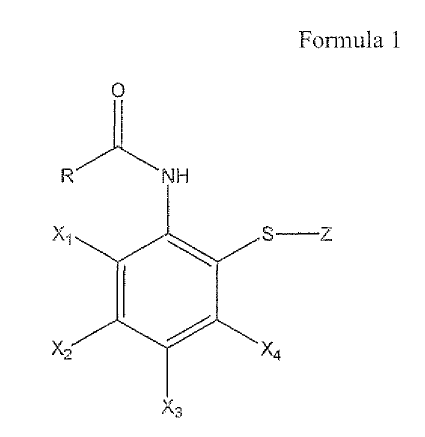 or a prodrug compound, pharmaceutically acceptable salt, enantiomer, stereoisomer, hydrate, or solvate thereof, in which
R represents
a substituted or unsubstituted C₃₋₁₀ cycloalkyl group or a substituted or unsubstituted C₅₋₈ cycloalkenyl group;
each of X₁, X₂, X₃, and X₄ may be the same or different and represents
a hydrogen atom;
a halogen atom;
a C₁₋₄ alkyl group;
a halo-C₁₋₄ alkyl group;
a C₁₋₄ alkoxy group;
a cyano group;
a nitro group;
an acyl group; or
an aryl group; and
Z represents
a hydrogen atom;
-YR₁, wherein
Y represents -CO- or -CS-, and
R₁ represents
a substituted or unsubstituted straight chain or branched C₁₋₁₀ alkyl group;
a C₁₋₄ alkoxy group;
a C₁₋₄ alkylthio group;
a substituted or unsubstituted amino group;
a substituted or unsubstituted ureido group;
a substituted or unsubstituted C₃₋₁₀ cycloallcyl group;
a substituted or unsubstituted C₃₋₁₀ cycloalkyl C₁₋₁₀ alkyl group;
a substituted or unsubstituted aryl group;
a substituted or unsubstituted aralkyl group;
a substituted or unsubstituted arylalkenyl group;
a substituted or unsubstituted arylthio group;
a substituted or unsubstituted 5-or 6-membered heterocyclic group having 1-3 nitrogen, oxygen, or sulfur atoms; or
a substituted or unsubstituted 5-or 6-membered heteroarylakyl group; or
S R₂, wherein
R₂ represents
a substituted or unsubstituted C₁₋₄ alkyl group or
a substituted or unsubstituted aryl group.

3. The composition of claim 2, wherein the water-insoluble concentration-enhancing additive is crospovidone.

4. The composition of claim 2, wherein the cholesteryl ester transfer protein inhibitor is 8-[2-([[1-(2-ethylbutyl)cyclohexyl]carbonyl]amino)phenyl] 2-methylpropanethioate.

5. The composition of claim 4, wherein the cholesterol ester transfer protein inhibitor and the water-insoluble concentration-enhancing additive are in a weight ratio of about 2:1 to about 9:1.

6. The composition of claim 5, wherein the water-insoluble concentration-enhancing additive is crospovidone.

7. A pharmaceutical composition of any one of claims 1 to 6 for use in the treatment or prophylaxis of a cardiovascular disorder.

8. The pharmaceutical composition for use of claim 7, wherein the cardiovascular disorder is selected from the group consisting of atherosclerosis, peripheral vascular disease, dyslipidemia, hyperbetalipoproteinemnia, hypoalphalipoproteinemia, hypercholesterolemia, hypertriglyceridemia, familial-hypercholesterolemia, angina, ischemia, cardiac ischemia, stroke, myocardial infarction, reperfusion injury, angioplastic restenosis, hypertension, and vascular complications of diabetes, obesity or endotoxemia.

9. The pharmaceutical composition for use of claim 7, wherein the cardiovascular disorder is selected from the group consisting of cardiovascular disease, coronary heart disease, coronary artery disease, hypoalphalipoproteinemia, hyperbetalipoproteinemia, hypercholesterolemia, hyperlipidemia, atherosclerosis, hypertension, hypertriglyceridemia, hyperlipidoproteinemia, peripheral vascular disease, angina, ischemia, and myocardial infarction.

10. The pharmaceutical composition for use of claim 7, wherein a maximum concentration of the cholesteryl ester transfer protein inhibitor, or active form thereof, in the bloodstream of a mammal is at least about 0.35 µg/mL post-treatment relative to pretreatment when the cholesteryl ester transfer protein inhibitor is *S*-[2-([[1-(2-ethylbutyl)cyclohexyl]carbonyl]-amino)phenyl] 2-methylpropanethioate administered at a daily dose of 600 mg with food.

11. The pharmaceutical composition for use of claim 7, wherein a maximum concentration of the cholesteryl ester transfer protein inhibitor, or active form thereof, in the bloodstream of a mammal is at least about 0.8 µg/mL post-treatment relative to pretreatment when the cholesterol ester transfer protein inhibitor is *S-*[2-([[1-(2-ethylbutyl)cyclohexyl]carbonyl]-amino)phenyl]2-methylpropanethioate administered at a daily dose of 900 mg with food.

12. The pharmaceutical composition for use of claim 7, wherein an area under the plasma concentration time curve AUC_{0-∞} of the cholesteryl ester transfer protein inhibitor, or active form thereof, in the bloodstream of a mammal is at least about 3.5 µg·h/mL post-treatment relative to pretreatment when the cholesteryl ester transfer protein inhibitor is *S-*[2-([[1-(-ethylbutyl)cyclohexyl]carbonyl]amino)phenyl] 2-methylpropanethioate administered at a daily dose of 600 mg with food.

13. The pharmaceutical composition for use of claim 7, wherein an area under the plasma concentration time curve AUC_{0-∞} of the cholesteryl ester transfer protein inhibitor, or active form thereof, in the bloodstream of a mammal is at least about 7.5 µg·h/mL post-treatment relative to pretreatment when the cholesteryl ester transfer protein inhibitor is *S-*[2-([[1-(2-ethylbutyl)cyclohexyl]carbonyl]amino)phenyl] 2-methylpropanethioate administered at a daily dose of 900 mg with food.

14. The Pharmaceutical composition for use of claim 7, wherein cholesteryl ester transfer protein activity in the bloodstream of a mammal is inhibited post-treatment by at least about 25% relative to CETP activity pretreatment when the cholesteryl ester transfer protein inhibitor is 5'-[2-([[1-(2-ethylbutyl)cyclohexyl]carbonyl]amino)phenyl] 2-methylpropanethioate administered at a daily dose of 600 mg with food.

15. The pharmaceutical composition for use of claim 7, wherein cholesteryl ester transfer protein activity in the bloodstream of a mammal is inhibited post-treatment by at least about 35% relative to CETP activity pretreatment when the cholesteryl ester transfer protein inhibitor is *S*-[2-([[1-(2-ethylbutyl)cyclohexyl]carbonyl]amino)phenyl] 2-methylpropanethioate administered at a daily dose of 900 mg with food.
